# EUROPEAN PATENT SPECIFICATION

(11) **EP 4 004 926 B1**
(45) Date of publication and mention of the grant of the patent: **18.11.2026**
(21) Application number: 20751220.3
(22) Date of filing: 22.07.2020
(51) Int. Cl.: G16B 20/10

(54) **SYSTEM AND METHOD FOR COPY NUMBER VARIANT ERROR CORRECTION**
SYSTEM UND VERFAHREN ZUR FEHLERKORREKTUR VON KOPIENZAHLVARIANTEN
SYSTÈME ET PROCÉDÉ DE CORRECTION D'ERREUR VARIANTE DE NUMÉRO DE COPIE

(30) Priority: 22.07.2019 GB 201910478; 04.11.2019 GB 201916002
(43) Date of publication of application: 01.06.2022
(73) Proprietor: Congenica Ltd., Cambridge, Cambridgeshire CB4 0DP (GB)
(72) Inventor: RAYNER, Timothy, Cambridge Cambridgeshire CB4 0DP (GB); FOX, Peter, Cambridge Cambridgeshire CB4 0DP (GB); DRURY, Suzanne, Cambridge Cambridgeshire CB4 0DP (GB); LENCH, Nicholas, Cambridge Cambridgeshire CB4 0DP (GB)
(74) Representative: Crowell & Moring U.K. LLP
(86) International application number: PCT/GB2020/051753
(87) International publication number: WO 2021/014155

(56) References cited:
- EP-A1- 3 298 523
- WO-A1-2014/015319
- WO-A1-2015/148776
- WO-A1-2016/187051
- US-A1- 2014 235 456
- CHIANG T ET AL: "Atlas-CNV: a validated approach to call single-exon CNVs in the eMERGESeq gene panel", GENETICS IN MEDICINE, vol. 21, 1 January 2019 (2019-01-01), pages 2135 - 2144, XP055735985, DOI: 10.1038/s41436-

## Description

### TECHNICAL FIELD

The present disclosure relates generally to genomics; more specifically, the present disclosure relates to systems for copy number variant error correction, for example involving management of reference panels used for detecting copy number variants in a given genomic sequence. The present disclosure further relates to methods for correcting copy number variation errors, for example including management of reference panels used for detecting copy number variants in a given genomic sequence.

### BACKGROUND

With recent advancements in medical and computational technology, there has been a rapid progress in respect of genomic sequencing to generate corresponding sequencing data, and analysis of the corresponding sequencing data. The sequencing data is commonly generated in short-read sequences; for example, the short-read sequences are between 50 and 300 deoxyribonucleic acid (DNA) bases long. Moreover, these short-read sequences are distributed stochastically across a given patient's genome. Analysis of such sequencing data forms a basis for detecting certain features present in the given patient's genome, such as copy number variants (CNVs). By detecting such variants in the given patient's genome, ailments or abnormalities in the genome can be identified, that potentially facilitates a subsequent treatment of the identified ailments or abnormalities, for example by performing gene therapy.

Typically, detecting such variants in a genomic sequence of a given individual requires analysis of the genomic sequence of a given individual (i.e. a target sequence) with respect to a reference panel comprising one or more reference sequences. Currently, there are many major technical problems associated with conventional systems that use the reference panel for genomic analysis purposes. One of the major technical problems is that conventional systems and analysis methods for detection such variants, particularly CNV, in a genomic sequence using reference panel do not fit various laboratory workflows (i.e. end-user workflows). Alternatively stated, data management tasks related to a reference panel are complicated, and existing systems that employ a reference panel are not suitable for different workflows employed at different end-user entities. In many cases, end-user entities (e.g. laboratories) desire to use samples in a reference panel which have been processed through a same sequencing run as a target sample, where CNV analysis is to be carried out. In other cases, the end-user entities desire to use samples from previous runs that have been constructed into a standard reference panel. Existing solutions require the end-user to process manually each CNV request, which also includes manual assembly of all data required for a target sequence in which a CNV is to be detected and a reference panel, which is not only time consuming but also error prone. For example, a given sample is processed using many different laboratory techniques, such as whole genome sequencing, exome sequencing and so forth, to derive corresponding short-read sequences. Differences in types of sequencing used for deriving the genomic sequences introduce their own data errors or biases into the generated sequences. Thus, the comparison of a genomic sequence of the individual obtained from one type of sequencing, such as exome sequencing with the reference sequences obtained from another type of sequencing, such as whole genome sequencing may generate erroneous results in the detected variants (e.g. CNV) in the genomic sequence of the individual. Additionally, it is observed that there are many other factors that potentially affect the detection of variants, for example, sequencing equipment used, an origin of a sample, and the like. Such factors are generally unaccounted for in conventional systems. Subsequently, when detecting variants, especially CNV, using the abovementioned conventional systems and techniques, such detection is prone to errors and unreliability, wherein such error and unreliability leads to fallacious treatment procedures for ailments or abnormalities that are incorrectly identified. For example, currently rare disease patients, typically between 5 and 30 years old, are usually subjected to varying and potentially invasive diagnostic tests and such rare disease patients receive sub-optimal medical treatment due to misinterpretation of causative mutations. This sub-optimal medical treatment can result in an incorrect decision support for a physician to take precautionary measures, or treatment due to a missed assessment of a disease as a result of the misinterpretation of causative mutation (e.g. CNV) not being properly detected in a target sample of the given individual.

WO 2016/187051 A1 relates to methods and systems for determining copy number variants are disclosed. An example method can comprise applying a sample grouping technique to select reference coverage data, normalizing sample coverage data comprising a plurality of genomic regions, and fitting a mixture model to the normalized sample coverage data based on the selected reference coverage data. An example method can comprise identifying one or more copy number variants (CNVs) according to a Hidden Markov Model (HMM) based on the normalized sample coverage data and the fitted mixture model. An example method can comprise outputting the one or more copy number variants.

Therefore, in light of the foregoing discussion, there exists a need to overcome the aforementioned drawbacks associated with conventional system and method for management and use of reference panels, for example, for CNV detection, in the genomic sequence of the individual.

### SUMMARY

The present disclosure seeks to provide an improved system for managing a copy number variant (CNV) reference panel. The present disclosure also seeks to provide an improved method for (of) managing the CNV reference panel. The present disclosure seeks to provide a solution to an existing problem of complicated data management related to a reference panel which does not fit various laboratory workflows (i.e. end-user workflows). The present disclosure further seeks to provide a solution to an existing problem of uncertainty related to a selection of a reference panel (i.e. which reference panel to use and whether the used reference panel is appropriate for CNV calling task), resulting in improper analysis of a genomic sequence of an individual and unreliable detection of variants, such as CNV.

An aim of the present disclosure is to provide a solution that overcomes at least partially the problems encountered in prior art, and to provide a system and method that provides a solution that significantly simplifies data management related to a reference panel for high-throughput, automated genomic analysis, and provides a unified mechanism that is suitable for various laboratory workflows. The system reduces or almost removes uncertainty related to making a selection of an optimal reference panel and allows validation that the used reference panel is appropriate for the CNV calling task, thereby improving reliability of the system.

In one aspect, the present disclosure provides a system for detection of copy number variant, CNV, errors by using a customised reference panel, wherein the system comprises: a database arrangement that is configured to store a plurality of sample genomic DNA sequences and metadata that is associated with each of the plurality of sample genomic DNA sequences, wherein the plurality of sample genomic DNA sequences comprises pre-registered sequences generated from historic and recent sequencer runs; and a computing arrangement that is communicatively coupled to the database arrangement, wherein the computing arrangement is configured to:
acquire the plurality of sample genomic DNA sequences from the database arrangement;
retrieve a plurality of characteristic attributes related to the sample genomic DNA sequences to generate the metadata, wherein the plurality of characteristic attributes related to each of the sample genomic DNA sequences comprise information about:
   at least one protocol applied to derive a genomic DNA sequence wherein the at least one protocol comprises selecting:
   a type of sequencing for the derivation of the genomic DNA sequence;
   an area-of-genomic-interest for the derivation of the genomic DNA sequence;
   a type of sample used to derive the genomic DNA sequence;
   a gender of an individual from which the sample is acquired for the derivation of the genomic DNA sequence; and/or
   a familial record of the individual from which the sample is acquired for the derivation of the genomic DNA sequence;
tag the metadata that comprises the plurality of characteristic attributes with each of the plurality of sample genomic DNA sequences;
store the plurality of sample genomic DNA sequences and the associated metadata with each of the plurality of sample genomic DNA sequences in the database arrangement;
identify the sample genomic DNA sequences having same metadata from the plurality of sample genomic DNA sequences;
group the identified sample genomic DNA sequences having the same metadata into a common group;
store each group of identified sample genomic DNA sequences having the same metadata as one project of a plurality of projects;
tag each project of the plurality of projects with the metadata of the sample genomic DNA sequences present in that project, wherein the plurality of projects having the sample genomic DNA sequences forms a candidate reference panel;
render a user interface that is configured to receive a target genomic DNA sequence along with an interpretation request for calling CNVs in the target genomic DNA sequence, wherein the interpretation request comprises a plurality of characteristic attributes related to the target genomic DNA sequence;
compare the plurality of characteristic attributes in the interpretation request with the prestored metadata associated with each of the plurality of sample genomic DNA sequences in the database arrangement, comprising taking a record of the plurality of characteristic attributes in the interpretation request received from the user, retrieving the plurality of characteristic attributes in the metadata from the database arrangement and running a comparison with the plurality of characteristic attributes in the metadata;
utilise the candidate reference panel for selecting the customised reference panel for the target genomic DNA sequence, based on the comparison of the information in the interpretation request with the metadata of each sample genomic DNA sequence and a plurality of defined criteria that checks whether:
   at least one protocol applied to derive the sample genomic DNA sequence matches with the at least one protocol applied to derive the target genomic DNA sequence; and
utilise the customised reference panel comprising the identified set of sample genomic DNA sequences for calling CNVs in the target genomic DNA sequence, wherein the user interface is configured to allow submission of target genomic DNA sequence separately at a timepoint that is different from a timepoint when the reference panel is identified and specified for use as the reference panel for the target genomic DNA sequence.

In another aspect, the present disclosure provides a method for 30 detecting copy number variant, CNV, errors by using a reference panel, wherein the method is implemented using a system that comprises a database arrangement that is configured to store a plurality of sample genomic DNA sequences and metadata that is associated with each of the plurality of sample genomic DNA sequences, wherein the plurality of sample genomic DNA sequences comprises pre-registered sequences generated from historic and recent sequencer runs, and a computing arrangement that is communicatively coupled to the database arrangement (102), the method comprising:
acquiring, by use of the computing arrangement, the plurality of sample genomic DNA sequences from the database arrangement;
retrieving, by use of the computing arrangement, a plurality of characteristic attributes related to the sample genomic DNA sequences to generate the metadata, wherein the plurality of characteristic attributes related to each of the sample genomic DNA sequences comprise information about:
   at least one protocol applied to derive a genomic DNA sequence wherein the at least one protocol comprises selecting:
   a type of sequencing for the derivation of the genomic DNA sequence;
   an area-of-genomic-interest for the derivation of the genomic DNA sequence;
   a type of sample used for a derivation of the genomic DNA sequence;
   a gender of an individual from which the sample is acquired for the derivation of the genomic DNA sequence; and
   a familial record of the individual from which the sample is acquired for the derivation of the genomic DNA sequence;
tagging, by use of the computing arrangement, the metadata that comprises the plurality of characteristic attributes associated with each of the plurality of sample genomic DNA sequences; and
storing, by use of the computing arrangement, the plurality of sample genomic DNA sequences and the associated metadata with each of the plurality of sample genomic DNA sequences in the database arrangement;
identifying the sample genomic DNA sequences having same metadata from the plurality of sample genomic DNA sequences;
grouping the identified sample genomic DNA sequences having the same metadata into a common group;
storing each group of identified sample genomic DNA sequences having the same metadata as one project of a plurality of projects;
   tagging each project of the plurality of projects with the metadata of the sample genomic DNA sequences present in that project, wherein the plurality of projects having the sample genomic DNA sequences forms a candidate reference panel; and
rendering, by use of the computing arrangement, a user interface configured to receive a target genomic DNA sequence along with an interpretation request for calling CNVs in the target genomic DNA sequence, wherein the interpretation request comprises a plurality of characteristic attributes related to the target genomic DNA sequence;
comparing the plurality of characteristic attributes in the interpretation request with metadata associated with each of a
   plurality of sample genomic DNA sequences prestored in the database arrangement, comprising taking a record of the plurality of characteristic attributes in the interpretation request received from the user, retrieving the plurality of characteristic attributes in the metadata from the database arrangement and running a comparison with the plurality of characteristic attributes in the metadata;
utilising the candidate reference panel for selecting the customised reference panel for the target genomic DNA sequence, based on the comparison of the information in the interpretation request with the metadata of each sample genomic DNA sequence and a plurality of defined criteria that checks whether:
   at least one protocol applied to derive the sample genomic DNA sequence matches with the at least one protocol applied to derive the target genomic DNA sequence; and
utilizing the reference panel comprising the identified set of sample genomic DNA sequences for calling CNVs in the target genomic DNA sequence, wherein the user interface is configured to allow submission of target genomic DNA sequence separately at a timepoint that is different from a timepoint when the reference panel is identified and specified for use as the reference panel for the target genomic DNA sequence.

In yet another aspect, the present disclosure provides a computer program product comprising a non-transitory computer-readable storage medium having computer-readable instructions stored thereon, the computer-readable instructions being executable by a computerised device comprising processing hardware to execute the aforementioned method.

Embodiments of the present disclosure substantially eliminate, or at least partially address, the aforementioned problems in the prior art, and enables the system to identify automatically and accurately a set of sample genomic DNA sequence as a reference panel which is most suitable for a target genomic DNA sequence (exome) for the CNV calling task, thereby reducing or almost removing uncertainty related to selection of an optimal reference panel by an end-user; such uncertainty otherwise can give rise to errors. The identified set of sample genomic DNA sequence as a reference panel is not static for all target sequences, but are dynamic and different for different target genomic DNA sequences (exome). The present disclosure further addresses the data management issues related to reference panel for high throughput, automated genomic analysis, and provides a unified mechanism that is suitable for various laboratory workflows.

Additional aspects, advantages, features and objects of the present disclosure would be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those in the art will understand that the drawings are not to scale. Wherever possible, like elements have been indicated by identical numbers.

Embodiments of the present disclosure will now be described, by way of example only, with reference to the following diagrams wherein:
FIG. 1A is an illustration of a block diagram of a system for managing a copy number variant reference panel, in accordance with an embodiment of the present disclosure;
FIG. 1B is an illustration of a block diagram of a system for managing a copy number variant reference panel, in accordance with another embodiment of the present disclosure; and
FIG. 2 is a flowchart depicting steps of a method for managing a copy number variant reference panel, in accordance with an embodiment of the present disclosure.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item at which the arrow is pointing.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practicing the present disclosure are also possible.

In one aspect, the present disclosure provides a system for managing copy number variant (CNV) errors by using a reference panel, wherein the system comprises:
- a database arrangement that is configured to store a plurality of sample genomic DNA sequences and metadata that is associated with each of the plurality of sample genomic DNA sequences; and
- a computing arrangement that is communicatively coupled to the database arrangement, wherein the computing arrangement is configured to:
- render a user interface that is configured to receive a target genomic DNA sequence along with an interpretation request for calling CNVs in the target genomic DNA sequence, wherein the interpretation request comprises a plurality of characteristic attributes related to the target genomic DNA sequence;
- compare the plurality of characteristic attributes in the interpretation request with the prestored metadata associated with each of the plurality of sample genomic DNA sequences in the database arrangement;
- identify a set of sample genomic DNA sequences as a reference panel from the plurality of sample genomic DNA sequences, based on the comparison of the information in the interpretation request with the metadata of each sample genomic DNA sequence and a plurality of defined criteria; and
- utilise the reference panel comprising the identified set of sample genomic DNA sequences for calling CNVs in the target genomic DNA sequence, wherein the user interface is configured to allow submission of target genomic DNA sequence separately at a timepoint that is different from a timepoint when the reference panel is identified and specified for use as the reference panel for the target genomic DNA sequence.

In another aspect, the present disclosure provides a method for managing copy number variant (CNV) errors by using a reference panel, wherein the method is implemented using a system that comprises a database arrangement and a computing arrangement, the method comprising:
- rendering, by use of the computing arrangement, a user interface configured to receive a target genomic DNA sequence along with an interpretation request for calling CNVs in the target genomic DNA sequence, wherein the interpretation request comprises a plurality of characteristic attributes related to the target genomic DNA sequence;
- comparing the plurality of characteristic attributes in the interpretation request with metadata associated with each of a plurality of sample genomic DNA sequences prestored in the database arrangement;
- identifying a set of sample genomic DNA sequence as a reference panel from the plurality of sample genomic DNA sequences, based on the comparison of the information in the interpretation request with the metadata of each sample genomic DNA sequence and a plurality of defined criteria; and
- utilizing the reference panel comprising the identified set of sample genomic DNA sequences for calling CNVs in the target genomic DNA sequence, wherein the user interface is configured to allow submission of target genomic DNA sequence separately at a timepoint that is different from a timepoint when the reference panel is identified and specified for use as the reference panel for the target genomic DNA sequence.

In yet another aspect, the present disclosure provides a computer program product comprising a non-transitory computer-readable storage medium having computer-readable instructions stored thereon, the computer-readable instructions being executable by a computerised device comprising processing hardware to execute the aforesaid method.

The aforesaid system and method significantly simplify data management tasks related to a reference panel for high throughput, automated genomic analysis, and provides a unified mechanism that is suitable for various laboratory workflows. The present disclosure provides a mechanism (e.g. the user interface) through which each of the plurality of sample genomic DNA sequences is tagged with metadata. The metadata comprises information about a protocol that is applied to derive a genomic DNA sequence, i.e. a type of sequencing used, an area-of-genomic-interest, a type of sample used, a gender of an individual from which the sample is acquired, and a familial record of the individual from which the sample is acquired to derive the genomic DNA sequence. Such information provided in the metadata provides an insight into parameters, such as identity, quality and origin of the sample genomic DNA sequences that potentially forms a part of candidate reference panel.

Furthermore, the information in the metadata of the plurality of sample genomic DNA sequences is compared to information provided with the target genomic DNA sequence. Such comparison allows generation of a reference panel that dynamically comprises the set of sample genomic DNA sequences (e.g. at least 10 sample genomic DNA sequences) from the plurality of sample genomic DNA sequences. This reference panel comprising the set of sample genomic DNA sequences is customized and specifically for the target genomic DNA sequence in which CNV is to be detected. Alternatively stated, the system enables to identify automatically and accurately the set of sample genomic DNA sequence as a reference panel which is most suitable for the target genomic DNA sequence for the CNV calling task, thereby reducing or almost removing uncertainty related to selection of an optimal reference panel by an end-user; the uncertainty otherwise gives rise to potential errors. In other words, the identified set of sample genomic DNA sequence as a reference panel are not static for all target sequences, but are dynamic, i.e. different set of sample genomic DNA sequence are automatically and accurately identified as best (most suited) reference panel for different target genomic DNA sequences (exome) for CNV calling based on the comparison and the aforementioned plurality of criteria.

Furthermore, the system allows either pre-registered samples from historical sequencer runs or new samples from the current sequencer run to be used for the reference panel, and thus provides a comprehensive reference panel. The system enables submission of a genomic DNA sequence of a patient for analysis at a timepoint that is different from a timepoint of defining of the reference panels. By separating the sample submission and provision of the CNV reference panel, the system allows the reference panel to be submitted as a series of references, thereby simplifying the data management task and allowing a validation that the submitted reference panel is appropriate for the CNV calling task.

The disclosed system enables automatic generation of the reference panel, whereas in conventional systems, a user is required to manually process each CNV request, which also includes manual assembly of all data required for a target sequence in which CNV is to be detected and a reference panel, which is not only time consuming but also error prone. Thus, the system eliminates the errors caused in the detection of variants in the target genomic DNA sequence due to difference in sequences, types of samples, and the like and is able to detect the variants, especially CNV, in the target genomic DNA sequence with relatively high accuracy and reliability. Consequently, the system ensures a practical and highly accurate decision support for a physician to take precautionary measures, or treatment as a result of the accurate interpretation of causative mutation (e.g. CNV) that is properly detected in a target sample of a given individual. The system therefore provides a reduction in errors when performing DNA sequencing of samples, especially is respect of CNVs.

The present disclosure provides a system for managing a copy number variant (CNV) reference panel, wherein the system comprises a database arrangement that is configured to store a plurality of sample genomic DNA sequences and metadata that is associated with each of the plurality of sample genomic DNA sequences. The term "*copy number variant*" or CNV refers to sections of genome of an individual that are repeated and the number of repeats in the genome varies between individuals in the human population. The "*copy number variant*" is a result of copy number variation event, which is a type of duplication or deletion event that affects a considerable number of base pairs. Typically, differences in the DNA sequence in genomes contribute to uniqueness of an individual. These differences potentially influence most traits including susceptibility to disease. Since CNVs often encompass genes, the detection of CNVs have important roles both in human disease and drug response. Moreover, in comparison to other genetic variants (e.g. SNPs), CNVs are larger in size and can often involve complex repetitive DNA sequences. In certain cases, CNVs also encompass entire genes, which have a specific protein encoding function ascribed to them. For these reasons, CNVs are potentially more amenable to misinterpretation, and are difficult to detect as compared to other genetic variants.

It will be appreciated that the CNVs are linked with genetic disorders, such as genetic diseases and the like. In human genome, currently most CNVs are found to be benign variants that do not directly cause disease. However, there are several instances where CNVs that affect critical developmental genes and cause rare diseases. For example, there are certain reports of CNVs affecting nervous system, and contributing to Parkinson's Disease and Alzheimer's Disease. There could be thousands more CNVs in the human population, which lie undetected due to various reasons and problems discussed above. Thus, the system is configured to manage the CNV reference panel used for detection of the CNVs.

The term "*database arrangement*" refers to an organized body of digital information regardless of a manner in which its data or its organized body thereof is represented. Optionally, the database arrangement includes hardware, software, firmware and/or any combination thereof. For example, optionally, the organized body of related data is in the form of a table, a map, a grid, a packet, a datagram, a file, a document, a list or in any other form. The database arrangement includes any data storage software and systems, such as, for example, a relational database like IBM DB2 and Oracle. Optionally, the database arrangement is potentially used interchangeably herein as a database management system, as is common in the art. Furthermore, the database management system potentially includes software programs or applications to create and manage one or more databases. Optionally, the database arrangement is operable to support relational operations, regardless of whether it enforces strict adherence to a given relational model, as understood by those of ordinary skill in the art. Additionally, the database arrangement is populated by data elements. Furthermore, the data elements optionally include data records, bits of data, cells, that are used interchangeably herein and all intended to mean information stored in cells of the database arrangement. The database arrangement is configured to store the plurality of sample genomic DNA sequences derived from the genome or a portion of the genome of the individuals. The genomic DNA sequence represents an order of bases in the DNA, known as nucleotides, namely Adenine (A), Guanine (G), Cytosine (C) and Thymine (T) in pairs such that 'A' pairs with 'T' (A-T) and 'C' pairs with 'G' (C-G). The metadata associated with the plurality of sample genomic DNA sequences comprises information about the plurality of sample genomic DNA sequences that is stored in the database arrangement.

Moreover, the system comprises a computing arrangement that is communicatively coupled to the database arrangement. The term "*computing arrangement*" refers to a structure and/or hardware module that includes programmable and/or non-programmable components that are configured to store, process and/or share the biological information, such as the genomic DNA sequences related to the genome of the subject. Moreover, it will be appreciated that the computing arrangement is optionally implemented as a single hardware computing device, such as a server, or plurality of hardware computing devices operating in a parallel or distributed architecture. In an example, the computing arrangement optionally includes components such as a data memory device, a processor, a display, a network interface and the like, to store, process and/or share information with other computing components, such as a user device/user equipment/user interface. Examples of the computing arrangement include, but are not limited to, a medical system, a server, an electronic device, a specialized computational biology equipment, or other computing device. Optionally, the computing arrangement is part of a machine. The computing arrangement is communicatively coupled to the database arrangement, such as to retrieve the plurality of sample genomic DNA sequences and the metadata associated therewith from the database arrangement.

In an example embodiment, the computing arrangement is further configured to acquire the plurality of sample genomic DNA sequences from the database arrangement. The plurality of sample genomic DNA sequences comprises pre-registered sequences that are generated from historic sequencer runs and also new sequences that are generated by current (namely, recent) sequencer runs. In an example, in order to execute next generation sequencing (NGS), an input sample, such as a sample of DNA of a subject, is isolated from the subject. For example, after sampling blood, a small amount of DNA is isolated from the sampled blood. The quantity of isolated DNA is insufficient for sequencing library preparation. Therefore, the input sample is then fragmented into short sections. The length of these sections is optionally same, for example, less than 250 base pairs, optionally in a range of 100 to 250 base pairs. The length optionally also depends on a type of sequencing machine used or a type of experiment to be conducted. In some cases where the length of DNA sections is relatively longer, for example longer than 250 base pairs, the fragments are ligated with generic adaptors (i.e. small piece of known DNA located at the read extremities) and annealed to a glass slide using the adaptors (e.g. in Illumina based sequencing). In some cases, mRNA transcripts are isolated which correspond to the coding regions of functional genes, for example when performing exome sequencing.

In an example, in NGS, vast numbers of short reads (e.g. the plurality of cDNA fragment molecules) are sequenced in a single run. After the sequencing library is prepared, PCR is carried out to amplify each read, creating a spot with many copies of the same read. The amplified copies are then separated into single strands by denaturation for subsequent sequencing. In NSG, the sequencing is done in a parallel manner using sequencing-by-synthesis, to produce a set of concurrent data, composed of millions of short sequencing reads. The readout of the sequence by the system corresponds to the plurality of sample genomic DNA sequences (or readout). Thus, the database arrangement over a period of time includes pre-registered sequences that are generated from historical sequencer runs and also new sequences that are generated by current (namely, recent) sequencer runs.

The computing arrangement is further configured to retrieve a plurality of characteristic attributes related to the sample genomic DNA sequences to generate metadata, wherein the plurality of characteristic attributes related to each of the sample genomic DNA sequence comprises at least one protocol applied to derive a genomic DNA sequence: a type of sequencing, an area-of-genomic-interest. The plurality of characteristic attributes are features related to the sample genomic DNA sequence, where the features represent a plurality of properties of the sample genomic DNA sequence. The plurality of characteristic attributes related to two or more sample genomic DNA sequences are potentially same. Moreover, at least one characteristic attribute of the plurality of characteristic attributes related to two or more sample genomic DNA sequences is potentially same.

Optionally, the plurality of sample genomic DNA sequences are derived using at least one protocol via a wet-laboratory arrangement. The wet-laboratory arrangement is typically a facility, clinic and/or a setup of instruments, equipment and/or devices used for extracting (invasive or non-invasive), collecting, processing, and analysing body fluid samples; collecting, processing, and analysing genetic material; amplifying, enriching, and processing genetic material; and analysing the genetic information received from the amplified genetic material to derive the genome of the individual to generate the plurality of sample genomic DNA sequences. Herein, the instruments, equipment, and/or devices optionally include, but are not limited to, centrifuge, ELISA, spectrophotometer, PCR, RT-PCR, High-Throughput-Screening (HTS) system, next generation sequencing systems, Microarray system, Ultrasound, genetic analyzer, deoxyribonucleic acid (DNA) sequencer and SNP analyzer. Notably, *in vitro* processing of the biological sample is performed for deriving the genome of the individual to generate the plurality of sample genomic DNA sequences. Typically, a standard pipeline process is executed in sequencing to process the biological sample extracted from the individual in the wet-laboratory arrangement *in vitro* to prepare a sequencing library, for example, a library comprising a plurality of complementary deoxyribonucleic acid (cDNA) fragment molecules. Moreover, the biological sample of the individual refers to a laboratory specimen taken, preferably non-invasively, by sampling under controlled environments, that is, gathered matter of an individual's tissue, fluid, or other material derived from the individual. Examples of the biological sample include, but are not limited to, blood, throat swabs, sputum, saliva, surgical drain fluids, Chorionic villus sampling (CVS), tissue biopsies, amniotic fluid, or a sample of a foetus, such as cell free foetal DNA.

According to an embodiment, the plurality of characteristic attributes related to each of the sample genomic DNA sequence comprises the type of sequencing and the area-of-genomic-interest, as elucidated above. The type of sequencing refers to an exome sequencing, a shallow whole genome sequencing (sWGS), a targeted gene sequencing (amplicon, gene panel), a whole-transcriptome sequencing, a gene expression profiling with mRNA-sequencing, or a targeted gene expression profiling. The term "exome" refers to complete sequence of all exons in protein-coding genes in the genome. The area-of-genomic-interest refers to an objective of an experiment to find CNV in certain regions of interest (e.g. a group of genes or gene panels) in a genome. Typically, whole genome sequence CNV calling methods do not need a reference panel. Thus, the disclosed system is suited for CNV calling for exomes, and thus filters out such sequences which are sequenced from different type of sequencing other than exome sequencing.

According to an embodiment, the plurality of characteristic attributes related to each of the sample genomic DNA sequence further comprises a type of sample used to derive the genomic DNA sequence. The sample, i.e. a biological sample of the individual that refers to a laboratory specimen of the individual is taken, preferably non-invasively by sampling under controlled environments. The types of sample that are susceptible to being used to derive the genomic DNA sequence are, for example, blood, throat swabs, sputum, saliva, surgical drain fluids, Chorionic villus sampling (CVS), tissue biopsies, amniotic fluid, or sample of foetus, such as cell free foetal DNA. The sample of foetus is used to identify variations in prenatal testing. For example, the detection of early-infantile epileptic encephalopathy (EIEE) is performed by using the sample of foetus. The EIEE is a rare neurological disorder characterized by seizures. It is observed that epilepsy, in a significant percentage of children, is wrongly identified and treated as gastro intestinal disorders. The genomic DNA sequence obtained from the sample of foetus is optionally used as a reference to identify variants in genome of foetus of an individual (or a couple) at elevated risk of having a child affected with one of, or a preselected set of, Mendelian conditions, thereby enabling consideration of alternative productive options and early intervention strategies.

According to an embodiment, the plurality of characteristic attributes related to each of the sample genomic DNA sequence further comprises a gender of an individual from which the sample is acquired to derive the genomic DNA sequence. The sample genomic DNA sequence is susceptible to being acquired by the individual that is, for example, a male, a female. Thus, the plurality of characteristic attributes comprises information about the gender of the individual. Notably, gender (also referred to as "sex") is relevant when CNVs are to be detected where inheritance pattern (e.g. of a gene) is different by gender (i.e. sex). For example, when CNVs are to be detected in a region of " Y" chromosome of "XY" chromosome, then gender is used as one characteristic attribute of the plurality of characteristic attributes. Alternatively stated, gender may not be relevant to find CNVs where inheritance pattern is not different by sex. Moreover, certain genetic disorders, such as genetic diseases are predominant in one gender than other genders. For example, a disease, namely primary biliary cirrhosis occurs predominantly in human females, whereas a disease, namely, primary sclerosing cholangitis occurs predominantly in human males. A medical treatment of the identified ailments or abnormalities in the individual largely depend upon the gender of the individual. Thus, it is useful to have information of the gender of the individual from which the sample is acquired to derive the genomic DNA sequence, which in turn is potentially used as one of sequences in the reference panel.

According to an embodiment, the plurality of characteristic attributes related to each of the sample genomic DNA sequence further comprises a familial record of the individual from which the sample is acquired to derive the genomic DNA sequence. The familial record of the individual refers to information related to biological inheritance of the individual from which the sample is acquired to obtain the genomic DNA sequence. For example, a familial record of an individual refers to a family of the individual, where the genomic DNA sequence is likely to share genes (or potentially genetically inherited disease) from parents. Thus, the metadata comprises information about the plurality of characteristic attributes that is the information related to protocol applied to derive a genomic DNA sequence, the type of sample used to derive the genomic DNA sequence, a gender of an individual from which the sample is acquired, and the familial record of the individual from which the sample is acquired to derive the genomic DNA sequence.

According to an embodiment, the computing arrangement is further configured to tag the metadata that comprises the plurality of characteristic attributes with each of the plurality of sample genomic DNA sequences. The metadata related to a sample genomic DNA sequence is tagged therewith. The metadata works (namely, functions) as a classification of each of the plurality of sample genomic DNA sequences, and thus simplifies to identify a sample genomic DNA sequences having desired characteristic attributes to be included in a reference panel for CNV detection in downstream processing.

According to an embodiment, the computing arrangement is further configured to store the plurality of sample genomic DNA sequences and the associated metadata with each of the plurality of sample genomic DNA sequences in the database arrangement. The plurality of sample genomic DNA sequences and the associated metadata are stored in an associative relationship with each other in the database arrangement. Optionally, the database arrangement potentially comprises hundreds or thousands of sample genomic DNA sequences over period of time. These sample genomic DNA sequences are pre-registered sequences acquired from previous historical sequence runs as well as the new sequences acquired from the current sequence runs. More optionally, the plurality of sample genomic DNA sequences are updated as per requirements. In an example, the plurality of sample genomic DNA sequences comprises 100 sequences, such that the 100 sequences have corresponding metadata tagged therewith. The metadata associated with a first sequence (S1) is: whole genome sequencing (WGS) used as a sequencing technique, blood used as a type of sample, gender is female and S1 belongs to family A. The metadata associated with a second sequence (S2) is: exome sequencing used as a sequencing technique, saliva used as a type of sample, gender is male and S2 belongs to family B. The metadata associated with a third sequence (S3) is: WGS used as a sequencing technique, tissue used as a type of sample, gender is male and S3 belongs to family C. The metadata associated with a fourth sequence (S4) is: exome sequencing used as a sequencing technique, saliva used as a type of sample, gender is male and S4 belongs to family B. Similarly, other remaining sequences of the 100 sequences have corresponding metadata. Thus, the computing arrangement is configured to store the 100 sequences along with the metadata associated with them in the database arrangement.

According to an embodiment, the computing arrangement is further configured to identify sample genomic DNA sequences having same metadata from the plurality of sample genomic DNA sequences. The computing arrangement identifies the sample genomic DNA sequences from the plurality of sample genomic DNA sequences that have same metadata, i.e. the sample genomic DNA sequences are derived from a same sequencing technique, a type of sample used to derive sample genomic DNA sequences are same, a gender of the individual is same, but the familial record is different. For example, it is not desirable to have records from same family as if a child has gain or loss of genetic material then other family members are likely to suffer from same gain or loss, and so these may bias results and make loss or gain of CNVs look normal, which is actually not. Thus, having different familial record improves results of CNV detections by reducing biases. Referring to the abovementioned example, the computing arrangement identifies the sequences S2 and S4 as the genomic DNA sequences having compatible metadata (all characteristic attributes are same expect the familial record (i.e. different family).

According to an embodiment, the computing arrangement is further configured to group the identified sample genomic DNA sequences having the same metadata into a common group. Referring again to the abovementioned example, the computing arrangement groups the identified genomic DNA sequences S2 and S4 in a group as they have same metadata (i.e. compatible metadata) associated with them.

According to an embodiment, the computing arrangement is further configured to store each group of identified sample genomic DNA sequences having the same metadata as one project of a plurality of projects. The computing arrangement creates the plurality of projects, based on the similarity of the metadata associated with the plurality of sample genomic DNA sequences. Referring to the abovementioned example, the computing arrangement creates 3 projects. A first project includes a sequence S1 and the metadata associated with S1, a second project includes sequences S2 and S4 and the common metadata associated with S2 and S4, and a third project includes sequence S3 and the metadata associated with S3.

According to an embodiment, the computing arrangement is further configured to tag each project of the plurality of projects with the metadata of the sample genomic DNA sequences present in that project, wherein the plurality of projects having the sample genomic DNA sequences forms a candidate reference panel. Referring to the abovementioned example, the computing arrangement tags the metadata associated with the sequence S1 with the first project, the metadata associated with the sequences S2 and S4 with the second project and the metadata associated with the sequence S3 with the third project. The computing arrangement stores the tagged plurality of projects in the database arrangement such that the plurality of projects having the sample genomic DNA sequences forms a candidate reference panel. The candidate reference panel comprises all the sample genomic DNA sequences acquired from the historic sequencer runs and the current sequencer runs. The candidate reference panel is utilised for selecting a customised reference panel for the target genomic DNA sequence.

Furthermore, the computing arrangement is configured to render a user interface that is configured to receive a target genomic DNA sequence along with an interpretation request for calling CNVs in the target genomic DNA sequence, wherein the interpretation request comprises a plurality of characteristic attributes related to the target genomic DNA sequence. The term "user interface" refers to a graphical user interface having a structured set of user interface elements rendered on a display screen. Optionally, the user interface (UI) rendered on the display screen is generated by any collection or set of instructions executable by an associated digital system, such as the computing arrangement. Additionally, the user interface (UI) is operable to interact with a user to convey information such as graphical and/or textual information and receive input from the user. Furthermore, the user interface (UI) elements refer to visual objects that have a size and position in user interface (UI). A user interface element is, optionally, visible, although at times a user interface element is hidden. A user interface control is considered to be a user interface element. Text blocks, labels, text boxes, list boxes, lines, and images windows, dialog boxes, frames, panels, menus, buttons, icons, etc. are examples of user interface elements. In addition to size and position, a user interface element optionally has other properties, such as a margin, spacing, or the like. The computing arrangement is configured to render the user interface to receive the target genomic DNA sequence. The target genomic DNA sequence refers to a genomic DNA sequence in which the variants, such as the CNVs are to be detected. The target genomic DNA sequence is, for example, obtained by using the sequencing techniques utilised for deriving the plurality of sample genomic DNA sequences as explained above. For example, the target genomic DNA sequence is derived from exome sequencing or whole genome sequencing. The system is optionally used in end-user entities, such as genomics research centre, laboratories, sequencing centre and the like. The users at such locations utilise the user interface to provide (i.e. submit) the target genomic DNA sequence of an individual, for example a patient, to the computing arrangement. Notably, such locations are used to determine genomic data, such as variants in the genome of the patient to identify CNVs responsible for presence of the genetic disorders in the patient. The user inputs the target genomic DNA sequence along with the interpretation request, such that the interpretation request comprises information of the plurality of characteristic attributes related to the target genomic DNA sequence. Optionally, the user interface is potentially used to submit API (application programming interface) to be integrated with another data processing platform to perform the functionalities of the system. More optionally, the functionalities of the system are potentially operated by a command line interface (e.g. a command line client).

According to an embodiment, the plurality of characteristic attributes related to the plurality of sample genomic DNA sequences in the metadata and the plurality of characteristic attributes related to the target genomic DNA sequence in the interpretation request are mutually common. The plurality of characteristic attributes related to the target genomic DNA sequence are same as the plurality of characteristic attributes in the prestored metadata. The plurality of characteristic attributes stored in metadata includes at least one protocol applied to derive a genomic DNA sequence: a type of sequencing, an area-of-genomic-interest; the type of sample used to derive the genomic DNA sequence; the gender of the individual from which the sample is acquired to derive the genomic DNA sequence and the familial record of the individual from which the sample is acquired to derive the genomic DNA sequence. Optionally, the interpretation request comprises information other than the plurality of characteristic attributes in the metadata, for example, an age of the individual and patient ID and so forth. Optionally, the information in the interpretation request is stored in the database arrangement in a form of a table with links to one or more file formats, such as a binary alignment map (BAM) format, which is a binary format for storing the sequence data. More optionally, the file format is a FASTQ format, which is a text-based format for storing variant calls and corresponding information after the target genomic DNA sequencing is de-multiplexed. The FASTQ format (also referred to as Fastq) is a common format that is employed for storing next generation sequencing (NGS) data. The FASTQ format is a raw data file format. The files that are in FASTQ format are converted to files with a BAM format for processing.

Furthermore, the computing arrangement is configured to compare the plurality of characteristic attributes in the interpretation request with the prestored metadata associated with each of the plurality of sample genomic DNA sequences in the database arrangement. The computing arrangement takes a record of the plurality of characteristic attributes in the interpretation request received from the user, retrieves the plurality of characteristic attributes in the metadata from the database arrangement and runs a comparison with the plurality of characteristic attributes in the metadata.

Moreover, the computing arrangement is configured to identify a set of sample genomic DNA sequences as a reference panel from the plurality of sample genomic DNA sequences, based on the comparison of the information in the interpretation request with the metadata of each sample genomic DNA sequence and a plurality of defined criteria. The reference panel comprises the set of sample genomic DNA sequences that are used as the reference for determining the variants, such as the CNVs in the target genomic DNA sequence of the individual. The variants present in such references are potentially predetermined, thus are used as a ground truth for determining the variants present in the target genomic DNA sequence. The reference panel is selected based on prerequisite requirements specified in the interpretation request provided by the user. Optionally, the set of sample genomic DNA sequences comprises at least 10 sample genomic DNA sequences selected from thousands of sample genomic DNA sequences from diverse sources or projects.

According to an embodiment, the computing arrangement is configured to identify the set of sample genomic DNA sequences as the reference panel from the plurality of sample genomic DNA sequences based on the plurality of defined criteria that checks whether at least one protocol applied to derive the sample genomic DNA sequence matches with the at least one protocol applied to derive the target genomic DNA sequence. The computing arrangement identifies the sample genomic DNA sequence from the plurality of sample genomic DNA sequences as a part of the reference panel if the at least one protocol applied to derive the sample genomic DNA sequence, such as the type of sequencing and the area-of-genomic-interest is same as that of the protocol applied to derive the target genomic DNA sequence. The use of the sample genomic DNA sequence in which the same protocol is applied for deriving the sequence as the protocol applied for deriving the target genomic DNA sequence as the reference enables reduction in biases (namely, a reduction in errors) that potentially arise due to selection of a reference sequence derived from a different type of sequencing technique. For example, a sample genomic DNA sequence derived from WGS used as a reference for a target genomic DNA sequence derived from exome sequencing introduce biases in the results, thus it potentially detects false (namely, erroneous) variants in the target genomic DNA sequence. Thus, use of the sample genomic DNA sequence in which a same area-of-genomic-interest is used for deriving the sequence as that of the target genomic DNA sequence generates reliable detection (namely, provides error reduction) of variants in the target genomic DNA sequence. For example, in certain cases, a user wants to focus on a group of genes (gene panels) that potentially contribute to disease-causing phenotype. Having certain sample genomic DNA sequences in a reference panel with an area-of-genomic-interest where such group of genes (gene panels) are present is potentially beneficial for determining the CNVs in the target genomic DNA sequence that contribute to the disease-causing phenotype.

According to an embodiment, the computing arrangement is configured to identify the set of sample genomic DNA sequences as the reference panel from the plurality of sample genomic DNA sequences based on the plurality of defined criteria that further checks whether or not the type of sample used to derive the sample genomic DNA sequence matches with the type of sample used to derive the target genomic DNA sequence. The type of sample used in different sequencing runs are potentially different. For example, a quality of a sample genomic DNA sequence derived from the type of sample being blood is potentially different from a quality of a sample genomic DNA sequence derived from the type of sample being a cell free foetal DNA. In cases where the type of sample used to derive the sample genomic DNA sequence matches with the type of sample used to derive the target genomic DNA sequence, the reliability of CNV detection from such sample genomic DNA sequence (when used as a part of the reference panel) increases. The accurate detection of variants, particularly CNV, thus depends on the common type of sample used in target as well as reference panel.

According to an embodiment, the computing arrangement is configured to identify the set of sample genomic DNA sequences as the reference panel from the plurality of sample genomic DNA sequences based on the plurality of defined criteria that further checks whether or not the gender of the individual from which the sample for the sample genomic DNA sequence is acquired matches with the gender of the individual from which the sample for the target genomic DNA sequence is acquired. A given patient potentially requires a medical treatment for a genetic disorder that is gender-specific. Notably, certain genetic disorders are predominant in only females, whereas certain other genetic disorders are predominant in only males. Thus, for a female patient, a sample genomic DNA sequence from a female is preferably used as a reference to identify variants in the female patient that potentially have caused genetic disorders in that female patient. Similarly, for a male patient, a sample genomic DNA sequence from a male is preferably used as a reference to identify variants in the male patient that potentially have caused genetic disorders in that male patient.

According to an embodiment, the computing arrangement is further configured to record a gender of the individual from which a sample is acquired to derive the target genomic DNA sequence as female, if the gender of the individual is undisclosed in the interpretation request. Typically, a gender of the individual is specified in the interpretation request. In an example case, when the gender of the individual is not specified, the computing arrangement records the gender as female (for example, as a default).

According to an embodiment, the computing arrangement is configured to identify the set of sample genomic DNA sequences as the reference panel from the plurality of sample genomic DNA sequences based on the plurality of defined criteria that further checks whether or not the familial record of the individual from which the sample genomic DNA sequence is obtained, is different from the familial record of the individual from which the target genomic DNA sequence is obtained. A majority of base pairs in DNA sequences from a same given family generally matches, thus, the variants in the target genomic DNA sequence remains unidentified if the sample genomic DNA sequence used as the reference is taken from the same family as that of the target genomic DNA sequence. Therefore, for a specific target genomic DNA sequence, the reference panel comprises the set of sample genomic DNA sequences that are not from the same family as that of the target genomic DNA sequence. In an example, a target genomic DNA sequence is acquired from a cell free foetal DNA. The reference panel, at least for purposes of CNV detection for the target genomic DNA sequence, does not comprise the sample genomic DNA sequences of a father or a mother of the foetus from which the cell free foetal is acquired.

According to an embodiment, the computing arrangement is further configured to reject the interpretation request, if a number of sample genomic DNA sequences in the set of sample genomic DNA sequences identified as the reference panel is less than a specified threshold number of sample genomic DNA sequences. The specified threshold number of sample genomic DNA sequences refers to a minimum number of sample genomic DNA sequences that are sufficient to be used as references in the reference panel for identifying the CNVs in the target genomic DNA sequence. Optionally, the specified threshold number of sample genomic DNA sequences is 10. Thus, if the number of sample genomic DNA sequences in the set of sample genomic DNA sequences identified as the reference panel is less than the threshold number 10, the interpretation request made by the user for identifying the CNVs in the target genomic DNA sequence is rejected.

Furthermore, the computing arrangement is configured to utilise the reference panel comprising the identified set of sample genomic DNA sequences for calling CNVs in the target genomic DNA sequence, wherein the user interface is configured to allow submission of target genomic DNA sequence separately at a timepoint that is different from a timepoint when the reference panel is identified and specified for use as the reference panel for the target genomic DNA sequence. The set of sample genomic DNA sequences identified as the reference panel is utilised for calling variants, such as CNVs in the target genomic DNA sequence. The submission of target genomic DNA sequence separately at the timepoint that is different from the timepoint when the reference panel is identified allows for a simplification of data management task and further allows for a validation that the reference panel is suitable for calling CNVs in the target genomic DNA sequence.

According to an embodiment, the database arrangement is configured to store at least one CNV detection application, and wherein the computing arrangement is configured to utilise the CNV detection application for calling of CNVs in the target genomic DNA sequence. The term "CNV detection application" refers to different applications that, when executed by the computing arrangement, potentially detect CNVs in the target genomic DNA sequence. Optionally, the at least one CNV detection application is a software application, algorithm, or a plurality of executable codes. Examples of the at least one CNV detection application include, but are not limited to, regression-based CNV detection application, read depth data-based CNV detection application, and the like. An example of CNV detection application include "ExomeDepth". The ExomeDepth is a CNV detection application that uses comparison of read depth coverage to call CNVs from the target genomic DNA sequence. The at least one CNV detection application is stored in the database arrangement, such that the computing arrangement utilizes one or more stored CNV detection application to call CNVs in the target genomic DNA sequence. Generally, whole genome sequence CNV calling methods or applications do not need a reference panel. Thus, the disclosed system is suited for CNV calling applications (or algorithms) for exomes.

According to an embodiment, the computing arrangement is further configured to execute the CNV detection application to compare an aggregate read depth that corresponds to the set of sample genomic DNA sequences identified as the reference panel with a corresponding read depth of the target genomic DNA sequence to identify regions in the target genomic DNA sequence that overlap with the set of sample genomic DNA sequences, indicative of a sequence coverage above a threshold level. The aggregate read depth is the average read depth of the set of sample genomic DNA sequences that is compared with the read depth of the target genomic DNA sequence. The comparison helps in identifying regions in the target genomic DNA sequence where CNVs are likely to be detected. As mentioned before, CNVs are a sequence of nucleotides in the genomic DNA sequence, and thus, overlap of the sequence of nucleotides in the regions of target genomic DNA sequence with the sequence of nucleotides in the sample genomic DNA sequence helps identifying the CNVs in the target genomic DNA sequence. The "threshold level" refers to a minimum amount of overlap that indicates a presence of CNV in the target genomic DNA sequence. Thus, if the overlap of the sequence of nucleotides in the target genomic DNA sequence and the sequence of nucleotides in the set of sample genomic DNA sequences is more than the threshold level, the computing arrangement, with the help of the CNV detection application identifies a CNV in the target genomic DNA sequence. Optionally, the threshold level is at least 50% overlap of the sequence of nucleotides.

According to an embodiment, the computing arrangement is further configured to execute the CNV detection application to rank each sample genomic DNA sequence of the set of sample genomic DNA sequences in the reference panel, based on the identified regions in the target genomic DNA sequence that overlap with one or more portions of each of the set of sample genomic DNA sequences. The CNV detection application ranks each sample genomic DNA sequence based on the overlapping regions of the sample genomic DNA sequence and the target genomic DNA sequence. The CNV detection application assigns a higher rank to a sample genomic DNA sequence that has greater overlapping region than a sample genomic DNA sequence that has lesser overlapping region. For example, a sample genomic DNA sequence S1 shows 70% overlapping regions with the target genomic DNA sequence; a sample genomic DNA sequence S2 shows 43% overlapping regions with the target genomic DNA sequence; and a sample genomic DNA sequence S3 shows 85% overlapping regions with the target genomic DNA sequence. The CNV detection application assigns a first rank to S3, a second rank to S1 and a third rank to S2, such that the first rank is the highest and the second rank is higher than the third rank.

The computing arrangement is further configured to execute the CNV detection application to eliminate the sample genomic DNA sequence of the set of sample genomic DNA sequences from the reference panel having overlapping regions less than the threshold level. The CNV detection application eliminates the sample genomic DNA sequence that are unsuitable to be used as a reference in the reference panel and potentially lead to detection of false CNVs in the target genomic DNA sequence. Referring to the abovementioned example, the CNV detection application eliminates the sample genomic DNA sequence S2 from the reference panel as S2 has overlapping regions compared to the target genomic DNA sequence less than the threshold level, for example 50%; and may lead to detection of false CNVs in the target genomic DNA sequence.

According to an embodiment, the computing arrangement is further configured to execute the CNV detection application to generate a confidence score as a measure of accuracy in the calling of CNVs in the target genomic DNA sequence. It will be appreciated that, for the comparison to be reliable, the sample genomic DNA sequence should be highly correlated with the target genomic DNA sequence of the patient, in order to reduce (for example, minimise) the level of bias and technical variability and thus, promote making of high-confidence CNV calls in the sequence. Optionally, higher the confidence score, better is the reliability of the detected CNVs in the target genomic DNA sequence. For example, the confidence score of 10 is regarded as a score that indicates the detected of CNVs is reliable; the score is thus a measure of potential error risk.

According to an embodiment, the computing arrangement is further configured to display patient information via the user interface (UI), and wherein the patient information comprises at least patient overview information and variant information. The patient overview information comprises a status of the interpretation request, wherein the status of the interpretation request is any one of: pending, complete, rejected. The status of the interpretation request shows *pending,* when the computing arrangement is yet to generate results related to CNV detection in the target genomic DNA sequence of the patient. The status of the interpretation request shows *complete,* when the computing arrangement, with the help of CNV detection application, has detected CNVs in the target genomic DNA sequence of the patient. The status of the interpretation request shows *rejected,* when the number of sample genomic DNA sequences identified as the reference panel for detection of CNVs is less than a specified number of sample genomic DNA sequences.

According to an embodiment, the patient overview information further comprises a protocol applied to derive the target genomic DNA sequence of a patient. For example, the protocol applied to derive the target genomic DNA sequence of a patient is whole genome sequencing or exome sequencing. The computing arrangement displays the protocol related to the target genomic DNA sequence.

According to an embodiment, the patient overview information further comprises a type of sample that is utilised to derive the target genomic DNA sequence of the patient. The type of sample that is utilised to derive the target genomic DNA sequence of the patient is displayed by the computing arrangement on the user interface (UI). For example, the computing arrangement displays the type of sample utilised to derive the target genomic DNA sequence of the patient as blood.

According to an embodiment, the patient overview information further comprises a reference panel selected for calling CNVs in the target genomic DNA sequence when the interpretation request is accepted. The reference panel optionally comprise the set of sample genomic DNA sequences selected to be used as a reference for calling CNVs in the target genomic DNA sequence of the patient. Optionally, in case the interpretation request is rejected, the computing arrangement displays information regarding the insufficient correlation found between the set of sample genomic DNA sequences and the target genomic DNA sequence for validation purposes.

According to an embodiment, the variant information of a patient comprises CNV gain or CNV loss in the target genomic DNA sequence as compared to the set of genomic DNA sequences identified as the reference panel. The CNV gain refers to a number of additional CNVs observed in the target genomic DNA sequence compared to the set of sample genomic DNA sequences. The CNV loss refers to a number of CNVs not observed in the target genomic DNA sequence compared to the set of sample genomic DNA sequences. The CNV gain and CNV loss are calculated based on certain factors, such as reads expected, reads observed, and the like. The computing arrangement displays information regarding the reads expected, the reads observed, ratio of the reads, and the CNVs calculated by using the ratio of the reads. Notably, reads expected are the aggregate read depth of the set of sample genomic DNA sequences. The reads observed are the read depth of the target genomic DNA sequence. The ratio of the reads is the ratio of reads observed divided by reads expected.

According to an embodiment, the variant information of a patient further comprises a confidence score generated for the calling of CNVs in the target genomic DNA sequence. The computing arrangement displays the confidence score generated for the calling of CNVs in the target genomic DNA sequence as the measure of measure of accuracy in the calling of CNVs in the target genomic DNA sequence; such a measure of accuracy is an indication of a measure of error reduction that is achieved.

The present disclosure also relates to the method as described above. Various embodiments and variants disclosed above apply *mutatis mutandis* to the method.

According to an embodiment, the method further comprises:
- acquiring, by use of the computing arrangement, the plurality of sample genomic DNA sequences from the database arrangement;
- retrieving, by use of the computing arrangement, a plurality of characteristic attributes related to the sample genomic DNA sequences to generate metadata, wherein the plurality of characteristic attributes related to each of the sample genomic DNA sequence comprises:
   - at least one protocol applied to derive a genomic DNA sequence: a type of sequencing, an area-of-genomic-interest;
   - a type of sample used for a derivation of the genomic DNA sequence;
   - a gender of an individual from which the sample is acquired for the derivation of the genomic DNA sequence; and
   - a familial record of the individual from which the sample is acquired for the derivation of the genomic DNA sequence;
- tagging, by use of the computing arrangement, the metadata that comprises the plurality of characteristic attributes associated with each of the plurality of sample genomic DNA sequences; and
- storing, by use of the computing arrangement, the plurality of sample genomic DNA sequences and the associated metadata with each of the plurality of sample genomic DNA sequences in the database arrangement.

According to an embodiment, the method further comprises utilising, by use of the computing arrangement, a CNV detection application for calling of CNVs in the target genomic DNA sequence, and wherein at least one CNV detection application is stored in the database arrangement.

According to an embodiment, a computer program product comprising a non-transitory computer-readable storage medium having computer-readable instructions stored thereon, the computer-readable instructions being executable by a computerised device comprising processing hardware to execute a method as described above.

### DETAILED DESCRIPTION OF THE DRAWINGS

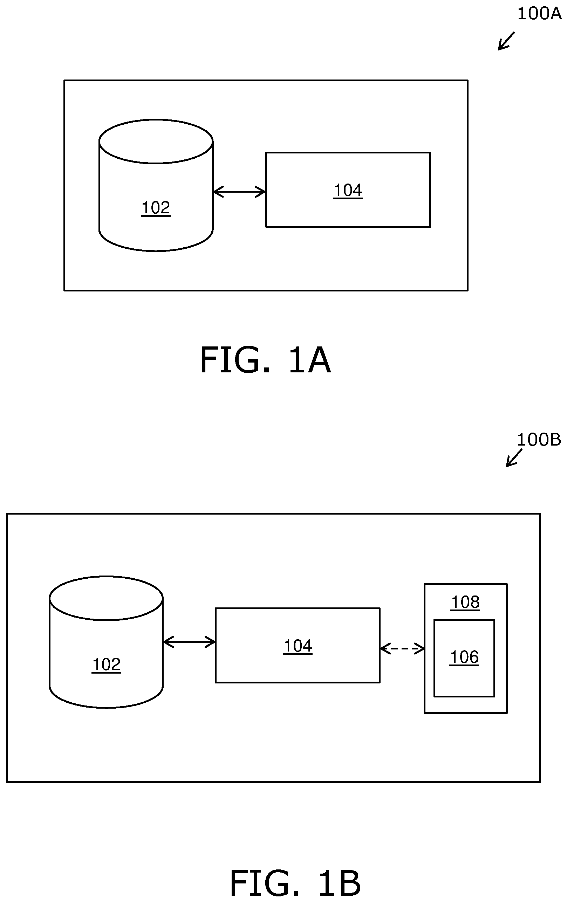

Referring to FIG. 1A, there is shown a block diagram of a system **100A** for managing a copy number variant reference panel, in accordance with an embodiment of the present disclosure. The system comprises a database arrangement **102** that is configured to store a plurality of sample genomic DNA sequences and metadata that is associated with each of the plurality of sample genomic DNA sequences. The system comprises a computing arrangement **104** that is communicatively coupled to the database arrangement **102.** The computing arrangement **104** is configured to render a user interface (not shown) that is configured to receive a target genomic DNA sequence along with an interpretation request for calling CNVs in the target genomic DNA sequence, wherein the interpretation request comprises a plurality of characteristic attributes related to the target genomic DNA sequence. Furthermore, the computing arrangement **104** is configured to compare the plurality of characteristic attributes in the interpretation request with the prestored metadata associated with each of the plurality of sample genomic DNA sequences in the database arrangement **102.** Moreover, the computing arrangement **104** is configured to identify a set of sample genomic DNA sequences as a reference panel from the plurality of sample genomic DNA sequences, based on the comparison of the information in the interpretation request with the metadata of each sample genomic DNA sequence and a plurality of defined criteria.

The computing arrangement **104** is further configured to utilise the reference panel comprising the identified set of sample genomic DNA sequences for calling CNVs in the target genomic DNA sequence, wherein the user interface is configured to allow submission of the target genomic DNA sequence separately at a timepoint that is different from a timepoint when the reference panel is identified and specified for use as the reference panel for the target genomic DNA sequence.

Referring to FIG. 1B, there is shown a block diagram of a system **100B** for managing a copy number variant reference panel, in accordance with another embodiment of the present disclosure. The system **100B** comprises a database arrangement **102.** The system **100B** further comprises a computing arrangement **104,** that is communicatively coupled to the database arrangement **102.** The computing arrangement **104** is configured to render a user interface **106** on a display device **108.** In this embodiment, the display device **108** is a separate device that is communicatively coupled to the computing arrangement **104.**

It is to be appreciated that, in some embodiments, the display device **108** is integrated to the computing arrangement **104.** In yet another embodiment, the computing arrangement **104** is a server, such that the server is configured to render remotely the user interface **106** on the display device **108.** It will be further appreciated by a person skilled in the art that the FIGs. 1A and 1B include a simplified illustration of the system **100A** and **100B** for sake of clarity only, which should not unduly limit the scope of the claims herein. The person skilled in the art will recognize many variations, alternatives, and modifications of embodiments of the present disclosure.

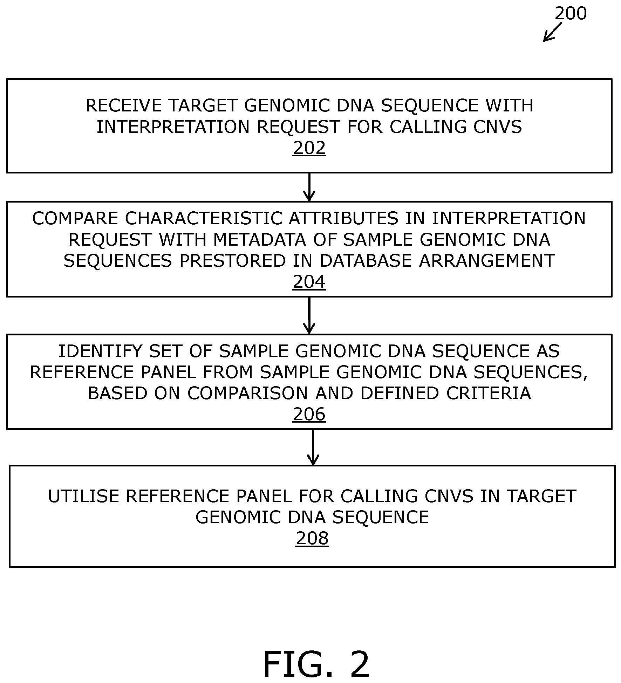

Referring next to FIG. 2, there is shown an illustration of a flowchart **200** depicting steps of a method for (of) managing a copy number variant (CNV) reference panel, in accordance with another embodiment of the present disclosure. As shown, at a step **202,** a target genomic DNA sequence along with an interpretation request for calling CNVs in the target genomic DNA sequence is received. The interpretation request comprises a plurality of characteristic attributes related to the target genomic DNA sequence. At a step **204,** the plurality of characteristic attributes in the interpretation request are compared with metadata associated with each of a plurality of sample genomic DNA sequences prestored in the database arrangement. At a step **206,** the set of sample genomic DNA sequence are identified as a reference panel from the plurality of sample genomic DNA sequences, based on the comparison of the information in the interpretation request with the metadata of each sample genomic DNA sequence and a plurality of defined criteria. At a step **208,** the reference panel comprising the identified set of sample genomic DNA sequences are utilised for calling CNVs in the target genomic DNA sequence, wherein the user interface is configured to allow submission of target genomic DNA sequence separately at a timepoint that is different from a timepoint when the reference panel is identified and specified for use as the reference panel for the target genomic DNA sequence.

The steps **202, 204, 206,** and **208** are only illustrative and other alternatives can also be provided where one or more steps are added, one or more steps are removed, or one or more steps are provided in a different sequence without departing from the scope of the claims herein.

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present.

## Claims

1. A system for detection of copy number variant, CNV, errors by using a customised reference panel, wherein the system comprises:
a database arrangement (102) that is configured to store a plurality of sample genomic DNA sequences and metadata that is associated with each of the plurality of sample genomic DNA sequences, wherein the plurality of sample genomic DNA sequences comprises pre-registered sequences generated from historic and recent sequencer runs; and
a computing arrangement (104) that is communicatively coupled to the database arrangement (102), wherein the computing arrangement (104) is configured to:
acquire the plurality of sample genomic DNA sequences from the database arrangement (102);
retrieve a plurality of characteristic attributes related to the sample genomic DNA sequences to generate the metadata, wherein the plurality of characteristic attributes related to each of the sample genomic DNA sequences comprises at least one protocol applied to derive a genomic DNA sequence, wherein the at least one protocol comprises selecting:
a type of sequencing for the derivation of the genomic DNA sequence;
an area-of-genomic-interest for the derivation of the genomic DNA sequence;
a type of sample used to derive the genomic DNA sequence;
a gender of an individual from which the sample is acquired for the derivation of the genomic DNA sequence; and/or
a familial record of the individual from which the sample is acquired for the derivation of the genomic DNA sequence;
tag the metadata that comprises the plurality of characteristic attributes with each of the plurality of sample genomic DNA sequences;
store the plurality of sample genomic DNA sequences and the associated metadata with each of the plurality of sample genomic DNA sequences in the database arrangement (102);
identify the sample genomic DNA sequences having same metadata from the plurality of sample genomic DNA sequences;
group the identified sample genomic DNA sequences having the same metadata into a common group;
store each group of identified sample genomic DNA sequences having the same metadata as one project of a plurality of projects;
tag each project of the plurality of projects with the metadata of the sample genomic DNA sequences present in that project, wherein the plurality of projects having the sample genomic DNA sequences forms a candidate reference panel;
render a user interface (106) that is configured to receive a target genomic DNA sequence along with an interpretation request for calling CNVs in the target genomic DNA sequence, wherein the interpretation request comprises a plurality of characteristic attributes related to the target genomic DNA sequence;
compare the plurality of characteristic attributes in the interpretation request with the prestored metadata associated with each of the plurality of sample genomic DNA sequences in the database arrangement (102), comprising taking a record of the plurality of characteristic attributes in the interpretation request received from the user, retrieving the plurality of characteristic attributes in the metadata from the database arrangement and running a comparison with the plurality of characteristic attributes in the metadata;
utilise the candidate reference panel for selecting the customised reference panel for the target genomic DNA sequence, based on the comparison of the information in the interpretation request with the metadata of each sample genomic DNA sequence and a plurality of defined criteria that checks whether at least one protocol applied to derive the sample genomic DNA sequence matches with the at least one protocol applied to derive the target genomic DNA sequence; and
utilise the customised reference panel comprising the identified set of sample genomic DNA sequences for calling CNVs in the target genomic DNA sequence, wherein the user interface (106) is configured to allow submission of target genomic DNA sequence separately at a timepoint that is different from a timepoint when the reference panel is identified and specified for use as the reference panel for the target genomic DNA sequence.

2. The system according to claim 1, wherein the plurality of characteristic attributes related to the plurality of sample genomic DNA sequences in the metadata and the plurality of characteristic attributes related to the target genomic DNA sequence in the interpretation request are mutually common.

3. The system according to any one of claims 1 and 2, wherein the computing arrangement (104) is further configured to reject the interpretation request, if a number of sample genomic DNA sequences in the set of sample genomic DNA sequences identified as the reference panel is less than a specified number of sample genomic DNA sequences.

4. The system according to any one of the preceding claims, wherein the computing arrangement (104) is further configured to record a gender of the individual from which a sample is acquired to derive the target genomic DNA sequence as female, if the gender of the individual is undisclosed in the interpretation request.

5. The system according to any one of the preceding claims, wherein the database arrangement (102) is configured to store at least one CNV detection application, and wherein the computing arrangement (104) is configured to utilize the CNV detection application for calling of CNVs in the target genomic DNA sequence.

6. The system according to claim 5, wherein the computing arrangement (104) is further configured to execute the CNV detection application to compare an aggregate read depth that corresponds to the set of sample genomic DNA sequences identified as the reference panel with a corresponding read depth of the target genomic DNA sequence to identify regions in the target genomic DNA sequence that overlap with the set of sample genomic DNA sequences, indicative of a sequence coverage above a threshold level.

7. The system according to claim 6, wherein the computing arrangement (104) is further configured to execute the CNV detection application to:
- rank each sample genomic DNA sequence of the set of sample genomic DNA sequences in the reference panel, based on the identified regions in the target genomic DNA sequence that overlap with one or more portions of each of the set of sample genomic DNA sequences; and
- eliminate the sample genomic DNA sequence of the set of sample genomic DNA sequences from the reference panel having overlapping regions less than the threshold level of 50%.

8. The system according to any one of claims 5 to 7, wherein the computing arrangement (104) is further configured to execute the CNV detection application to generate a confidence score as a measure of accuracy in the calling of CNVs in the target genomic DNA sequence.

9. The system according to any one of claims 2 to 8, wherein the computing arrangement (104) is further configured to display patient information via the user interface, and wherein the patient information comprises at least patient overview information and variant information, and wherein:
the patient overview information comprises:
a status of the interpretation request, wherein the status of the interpretation request is any one of: pending, complete, rejected;
a protocol applied to derive the target genomic DNA sequence of a patient;
a type of sample utilised to derive the target genomic DNA sequence of the patient; and
a reference panel selected for calling CNVs in the target genomic DNA sequence when the interpretation request is accepted; and
the variant information of a patient comprises:
CNV gain or CNV loss in the target genomic DNA sequence as compared to the set of genomic DNA sequences identified as the reference panel; and
confidence score generated for the calling of CNVs in the target genomic DNA sequence.

10. The system of any preceding claim, wherein checking whether at least one protocol applied to derive the sample genomic DNA sequence matches with the at least one protocol applied to derive the target genomic DNA sequence comprises checking whether:
the type of sample used to derive the sample genomic DNA sequence matches with the type of sample used to derive the target genomic DNA sequence;
the gender of the individual from which the sample for the sample genomic DNA sequence is acquired matches with the gender of the individual from which the sample for the target genomic DNA sequence is acquired; and/or
the familial record of the individual from which the sample genomic DNA sequence is obtained, is different from the familial record of the individual from which the target genomic DNA sequence is obtained.

11. A method for detecting copy number variant (CNV) errors by using a customised reference panel, wherein the method is implemented using a system that comprises a database arrangement (102) that is configured to store a plurality of sample genomic DNA sequences and metadata that is associated with each of the plurality of sample genomic DNA sequences, wherein the plurality of sample genomic DNA sequences comprises pre-registered sequences generated from historic and recent sequencer runs, and a computing arrangement (104) that is communicatively coupled to the database arrangement (102), the method comprising:
acquiring, by use of the computing arrangement (104), the plurality of sample genomic DNA sequences from the database arrangement (102);
retrieving, by use of the computing arrangement (104), a plurality of characteristic attributes related to the sample genomic DNA sequences to generate the metadata, wherein the plurality of characteristic attributes related to each of the sample genomic DNA sequences comprises at least one protocol applied to derive a genomic DNA sequence, wherein the at least one protocol comprises selecting:
a type of sequencing for the derivation of the genomic DNA sequence;
an area-of-genomic-interest for the derivation of the genomic DNA sequence;
a type of sample used for a derivation of the genomic DNA sequence;
a gender of an individual from which the sample is acquired for the derivation of the genomic DNA sequence; and
a familial record of the individual from which the sample is acquired for the derivation of the genomic DNA sequence;
tagging, by use of the computing arrangement (104), the metadata that comprises the plurality of characteristic attributes with each of the plurality of sample genomic DNA sequences;
storing, by use of the computing arrangement (104), the plurality of sample genomic DNA sequences and the associated metadata with each of the plurality of sample genomic DNA sequences in the database arrangement (102);
identifying the sample genomic DNA sequences having same metadata from the plurality of sample genomic DNA sequences;
grouping the identified sample genomic DNA sequences having the same metadata into a common group;
storing each group of identified sample genomic DNA sequences having the same metadata as one project of a plurality of projects;
tagging each project of the plurality of projects with the metadata of the sample genomic DNA sequences present in that project, wherein the plurality of projects having the sample genomic DNA sequences forms a candidate reference panel; and
rendering, by use of the computing arrangement (104), a user interface (106) configured to receive a target genomic DNA sequence along with an interpretation request for calling CNVs in the target genomic DNA sequence, wherein the interpretation request comprises a plurality of characteristic attributes related to the target genomic DNA sequence;
comparing the plurality of characteristic attributes in the interpretation request with metadata associated with each of a plurality of sample genomic DNA sequences prestored in the database arrangement (102), comprising taking a record of the plurality of characteristic attributes in the interpretation request received from the user, retrieving the plurality of characteristic attributes in the metadata from the database arrangement and running a comparison with the plurality of characteristic attributes in the metadata;
utilising the candidate reference panel for selecting the customised reference panel for the target genomic DNA sequence, based on the comparison of the information in the interpretation request with the metadata of each sample genomic DNA sequence and a plurality of defined criteria that checks whether at least one protocol applied to derive the sample genomic DNA sequence matches with the at least one protocol applied to derive the target genomic DNA sequence; and
utilising the reference panel comprising the identified set of sample genomic DNA sequences for calling CNVs in the target genomic DNA sequence, wherein the user interface (106) is configured to allow submission of target genomic DNA sequence separately at a timepoint that is different from a timepoint when the reference panel is identified and specified for use as the reference panel for the target genomic DNA sequence.

12. The method according to claim 11, wherein the method comprises utilising, by use of the computing arrangement (104), a CNV detection application for calling of CNVs in the target genomic DNA sequence, and wherein at least one CNV detection application is stored in the database arrangement (102).

13. The method of any of claims 11 or 12, wherein checking whether at least one protocol applied to derive the sample genomic DNA sequence matches with the at least one protocol applied to derive the target genomic DNA sequence comprises checking whether:
the type of sample used to derive the sample genomic DNA sequence matches with the type of sample used to derive the target genomic DNA sequence;
the gender of the individual from which the sample for the sample genomic DNA sequence is acquired matches with the gender of the individual from which the sample for the target genomic DNA sequence is acquired; and/or
the familial record of the individual from which the sample genomic DNA sequence is obtained, is different from the familial record of the individual from which the target genomic DNA sequence is obtained.

14. A computer program product comprising a non-transitory computer-readable storage medium having computer-readable instructions stored thereon, the computer-readable instructions being executable by a computerised device comprising processing hardware to execute a method as claimed in any one of claims 11 to 12. <--^{u}

## Patentansprüche

1. System zur Ermittlung von Fehlern bei Kopienzahlvarianten, CNV, durch Verwendung eines maßgeschneiderten Referenzpanels, wobei das System umfasst:
eine Datenbankanordnung (102), die dafür konfiguriert ist, eine Vielzahl von Proben-Genom-DNA-Sequenzen und Metadaten, die jeder der Vielzahl von Proben-Genom-DNA-Sequenzen zugeordnet sind, zu speichern, wobei die Vielzahl von Proben-Genom-DNA-Sequenzen vorab registrierte Sequenzen umfasst, die aus früheren und aktuellen Sequenziererläufen erzeugt wurden; und
eine Computeranordnung (104), die kommunikativ mit der Datenbankanordnung (102) gekoppelt ist, wobei die Computeranordnung (104) dafür konfiguriert ist:
die Vielzahl von Proben-Genom-DNA-Sequenzen aus der Datenbankanordnung (102) zu erlangen;
eine Vielzahl von charakteristischen Attributen abzurufen, die sich auf die Proben-Genom-DNA-Sequenzen beziehen, um die Metadaten zu erzeugen, wobei die Vielzahl von charakteristischen Attributen, die sich auf jede der Proben-Genom-DNA-Sequenzen beziehen, mindestens ein Protokoll umfasst, das angewendet wurde, um eine Genom-DNA-Sequenz abzuleiten, wobei das mindestens eine Protokoll Auswählen von Folgendem umfasst:
eine Art von Sequenzieren für die Ableitung der Genom-DNA-Sequenz;
einen Bereich von genomischem Interesse für die Ableitung der Genom-DNA-Sequenz;
eine Art der Probe, die verwendet wurde, um die Genom-DNA-Sequenz abzuleiten;
das Geschlecht einer Person, von der die Probe für die Ableitung der Genom-DNA-Sequenz erlangt wurde; und/oder
eine Familienanamnese der Person, von der die Probe für die Ableitung der Genom-DNA-Sequenz erlangt wurde;
die Metadaten, welche die Vielzahl von charakteristischen Attributen umfassen, mit jeder der Vielzahl von Proben-Genom-DNA-Sequenzen zu verknüpfen;
die Vielzahl von Proben-Genom-DNA-Sequenzen und die jeder der Vielzahl von Proben-Genom-DNA-Sequenzen zugeordneten Metadaten in der Datenbankanordnung (102) zu speichern;
diejenigen Proben-Genom-DNA-Sequenzen, die gleiche Metadaten aufweisen, aus der Vielzahl von Proben-Genom-DNA-Sequenzen zu identifizieren;
diejenigen identifizierten Proben-Genom-DNA-Sequenzen, welche die gleichen Metadaten aufweisen, zu einer gemeinsamen Gruppe zu gruppieren;
jede Gruppe von identifizierten Proben-Genom-DNA-Sequenzen, welche die gleichen Metadaten aufweisen, als ein Projekt einer Vielzahl von Projekten zu speichern;
jedes Projekt der Vielzahl von Projekten mit den Metadaten der in diesem Projekt vorliegenden Proben-Genom-DNA-Sequenzen zu verknüpfen, wobei die Vielzahl von Projekten, welche die Proben-Genom-DNA-Sequenzen aufweisen, ein Kandidaten-Referenzpanel bildet;
eine Benutzerschnittstelle (106) bereitzustellen, die dafür konfiguriert ist, eine Ziel-Genom-DNA-Sequenz zusammen mit einer Auswertungsanfrage zum Auffinden, auch Calling genannt, von CNVs in der Ziel-Genom-DNA-Sequenz zu empfangen, wobei die Auswertungsanfrage eine Vielzahl von charakteristischen Attributen umfasst, die sich auf die Ziel-Genom-DNA-Sequenz beziehen;
die Vielzahl von charakteristischen Attributen in der Auswertungsanfrage mit den vorab gespeicherten Metadaten zu vergleichen, die jeder der Vielzahl von Proben-Genom-DNA-Sequenzen in der Datenbankanordnung (102) zugeordnet sind, umfassend: Nehmen eines Datensatzes der Vielzahl von charakteristischen Attributen in der vom Benutzer empfangenen Auswertungsanfrage, Abrufen der Vielzahl von charakteristischen Attributen in den Metadaten aus der Datenbankanordnung und Durchführen eines Vergleichs mit der Vielzahl von charakteristischen Attributen in den Metadaten;
das Kandidaten-Referenzpanel zum Auswählen des maßgeschneiderten Referenzpanels für die Ziel-Genom-DNA-Sequenz zu nutzen, und zwar auf der Grundlage des Vergleichs der Informationen in der Auswertungsanfrage mit den Metadaten jeder Proben-Genom-DNA-Sequenz und einer Vielzahl von definierten Kriterien, die prüfen, ob mindestens ein Protokoll, das angewendet wurde, um die Proben-Genom-DNA-Sequenz abzuleiten, mit dem mindestens einen Protokoll übereinstimmt, das angewendet wurde, um die Ziel-Genom-DNA-Sequenz abzuleiten; und
das maßgeschneiderte Referenzpanel, das die identifizierte Menge von Proben-Genom-DNA-Sequenzen umfasst, zum Auffinden von CNVs in der Ziel-Genom-DNA-Sequenz zu nutzen, wobei die Benutzerschnittstelle (106) dafür konfiguriert ist, die Eingabe der Ziel-Genom-DNA-Sequenz separat zu einem Zeitpunkt zu ermöglichen, der sich von einem Zeitpunkt unterscheidet, zu dem das Referenzpanel identifiziert und zur Verwendung als Referenzpanel für die Ziel-Genom-DNA-Sequenz festgelegt wurde.

2. System nach Anspruch 1, wobei die Vielzahl von charakteristischen Attributen, die sich auf die Vielzahl von Proben-Genom-DNA-Sequenzen in den Metadaten beziehen, und die Vielzahl von charakteristischen Attributen, die sich auf die Ziel-Genom-DNA-Sequenz in der Auswertungsanfrage beziehen, miteinander übereinstimmen.

3. System nach einem der Ansprüche 1 und 2, wobei die Computeranordnung (104) ferner dafür konfiguriert ist, die Auswertungsanforderung abzulehnen, wenn eine Anzahl von Proben-Genom-DNA-Sequenzen in der als Referenzpanel identifizierten Menge von Proben-Genom-DNA-Sequenzen kleiner als eine festgelegte Anzahl von Proben-Genom-DNA-Sequenzen ist.

4. System nach einem der vorstehenden Ansprüche, wobei die Computeranordnung (104) ferner dafür konfiguriert ist, ein Geschlecht der Person, von der eine Probe erlangt wurde, um die Ziel-Genom-DNA-Sequenz abzuleiten, als weiblich zu protokollieren, wenn das Geschlecht der Person in der Auswertungsanfrage ungenannt ist.

5. System nach einem der vorstehenden Ansprüche, wobei die Datenbankanordnung (102) dafür konfiguriert ist, mindestens eine CNV-Ermittlungsanwendung zu speichern, und wobei die Computeranordnung (104) dafür konfiguriert ist, die CNV-Ermittlungsanwendung zum Auffinden von CNVs in der Ziel-Genom-DNA-Sequenz zu nutzen.

6. System nach Anspruch 5, wobei die Computeranordnung (104) ferner dafür konfiguriert ist, die CNV-Ermittlungsanwendung auszuführen, um eine aggregierte Lesetiefe, die der als Referenzpanel identifizierten Menge von Proben-Genom-DNA-Sequenzen entspricht, mit einer entsprechenden Lesetiefe der Ziel-Genom-DNA-Sequenz zu vergleichen, um Regionen in der Ziel-Genom-DNA-Sequenz zu identifizieren, die sich mit der Menge von Proben-Genom-DNA-Sequenzen überschneiden, was auf eine Sequenzabdeckung oberhalb eines Schwellenwerts hinweist.

7. System nach Anspruch 6, wobei die Computeranordnung (104) ferner dafür konfiguriert ist, die CNV-Ermittlungsanwendung auszuführen, um:
- jede Proben-Genom-DNA-Sequenz der Menge von Proben-Genom-DNA-Sequenzen in dem Referenzpanel auf der Grundlage der identifizierten Regionen in der Ziel-Genom-DNA-Sequenz, die sich mit einem oder mehreren Abschnitten einer jeden der Menge von Proben-Genom-DNA-Sequenzen überschneiden, zu ordnen; und
- die Proben-Genom-DNA-Sequenz der Menge von Proben-Genom-DNA-Sequenzen aus dem Referenzpanel zu entfernen, die überlappende Regionen unter dem Schwellenwert von 50 % aufweist.

8. System nach einem der Ansprüche 5 bis 7, wobei die Computeranordnung (104) ferner dafür konfiguriert ist, die CNV-Ermittlungsanwendung auszuführen, um einen Konfidenzwert als Maß für die Genauigkeit beim Auffinden von CNVs in der Ziel-Genom-DNA-Sequenz zu erzeugen.

9. System nach einem der Ansprüche 2 bis 8, wobei die Computeranordnung (104) ferner dafür konfiguriert ist, Patienteninformationen über die Benutzerschnittstelle anzuzeigen, und wobei die Patienteninformationen mindestens Patientenübersichtsinformationen und Varianteninformationen umfassen, und wobei:
die Patientenübersichtsinformationen umfassen:
einen Status der Auswertungsanfrage, wobei der Status der Auswertungsanfrage eines von Folgendem ist: ausstehend, abgeschlossen, abgelehnt;
ein Protokoll, das angewendet wurde, um die Ziel-Genom-DNA-Sequenz eines Patienten abzuleiten;
eine Art der Probe, die verwendet wurde, um die Genom-DNA-Sequenz des Patienten abzuleiten; und
ein Referenzpanel, das zum Auffinden von CNVs in der Ziel-Genom-DNA-Sequenz ausgewählt wurde, wenn die Auswertungsanfrage angenommen wurde; und
die Varianteninformationen eines Patienten umfassen:
einen CNV-Gewinn oder einen CNV-Verlust in der Ziel-Genom-DNA-Sequenz, verglichen mit der als Referenzpanel identifizierten Menge von Genom-DNA-Sequenzen; und
den Konfidenzwert, der für das Auffinden von CNVs in der Ziel-Genom-DNA-Sequenz erzeugt wurde.

10. System nach einem vorstehenden Anspruch, wobei Prüfen, ob mindestens ein Protokoll, das angewendet wurde, um die Proben-Genom-DNA-Sequenz abzuleiten, mit dem mindestens einen Protokoll übereinstimmt, das angewendet wurde, um die Ziel-Genom-DNA-Sequenz abzuleiten, umfasst: Prüfen, ob:
eine Art der Probe, die verwendet wurde, um die Proben-Genom-DNA-Sequenz abzuleiten, mit der Art der Probe übereinstimmt, die verwendet wurde, um die Ziel-DNA-Sequenz abzuleiten;
das Geschlecht der Person, von der die Probe für die Proben-Genom-DNA-Sequenz erlangt wurde, mit dem Geschlecht der Person übereinstimmt, von der die Probe für die Ziel-Genom-DNA-Sequenz erlangt wurde; und/oder
die Familienanamnese der Person, von der die Proben-Genom-DNA-Sequenz erlangt wurde, sich von der Familienanamnese der Person unterscheidet, von der die Ziel-Genom-DNA-Sequenz erhalten wurde.

11. Verfahren zur Ermittlung von Kopienzahlvarianten-(CNV-)Fehlern durch Verwendung eines maßgeschneiderten Referenzpanels, wobei das Verfahren unter Verwendung eines Systems durchgeführt wird, das eine Datenbankanordnung (102), die dafür konfiguriert ist, eine Vielzahl von Proben-Genom-DNA-Sequenzen und Metadaten, die jeder der Vielzahl von Proben-Genom-DNA-Sequenzen zugeordnet sind, zu speichern, wobei die Vielzahl von Proben-Genom-DNA-Sequenzen vorab registrierte Sequenzen umfasst, die aus früheren und aktuellen Sequenziererläufen erzeugt wurden, sowie eine Computeranordnung (104), die kommunikativ mit der Datenbankanordnung (102) gekoppelt ist, umfasst, wobei das Verfahren umfasst:
Erlangen der Vielzahl von Proben-Genom-DNA-Sequenzen aus der Datenbankanordnung (102) durch Verwendung der Computeranordnung (104);
Abrufen einer Vielzahl von charakteristischen Attributen, die sich auf die Proben-Genom-DNA-Sequenzen beziehen, durch Verwendung der Computeranordnung (104), um die Metadaten zu erzeugen, wobei die Vielzahl von charakteristischen Attributen, die sich auf jede der Proben-Genom-DNA-Sequenzen beziehen, mindestens ein Protokoll umfasst, das angewendet wurde, um eine Genom-DNA-Sequenz abzuleiten, wobei das mindestens eine Protokoll das Auswählen von Folgendem umfasst:
eine Art von Sequenzieren für die Ableitung der Genom-DNA-Sequenz;
einen Bereich von genomischem Interesse für die Ableitung der Genom-DNA-Sequenz;
eine Art der Probe, die für eine Ableitung der Genom-DNA-Sequenz verwendet wurde;
das Geschlecht einer Person, von der die Probe für die Ableitung der Genom-DNA-Sequenz erlangt wurde; und
eine Familienanamnese der Person, von der die Probe für die Ableitung der Genom-DNA-Sequenz erlangt wurde;
Verknüpfen der Metadaten, welche die Vielzahl von charakteristischen Attributen umfassen, mit jeder der Vielzahl von Proben-Genom-DNA-Sequenzen durch Verwendung der Computeranordnung (104);
Speichern der Vielzahl von Proben-Genom-DNA-Sequenzen und der jeder der Vielzahl von Proben-Genom-DNA-Sequenzen zugeordneten Metadaten in der Datenbankanordnung (102) durch Verwendung der Computeranordnung (104);
Identifizieren derjenigen Proben-Genom-DNA-Sequenzen, die gleiche Metadaten aufweisen, aus der Vielzahl von Proben-Genom-DNA-Sequenzen;
Gruppieren derjenigen identifizierten Proben-Genom-DNA-Sequenzen, welche die gleichen Metadaten aufweisen, zu einer gemeinsamen Gruppe;
Speichern jeder Gruppe von identifizierten Proben-Genom-DNA-Sequenzen, welche die gleichen Metadaten aufweisen, als ein Projekt einer Vielzahl von Projekten;
Verknüpfen jedes Projekts der Vielzahl von Projekten mit den Metadaten der in diesem Projekt vorliegenden Proben-Genom-DNA-Sequenzen, wobei die Vielzahl von Projekten, welche die Proben-Genom-DNA-Sequenzen aufweisen, ein Kandidaten-Referenzpanel bildet; und
Wiedergeben, durch Verwendung der Computeranordnung (104), einer Benutzerschnittstelle (106), die dafür konfiguriert ist, eine Ziel-Genom-DNA-Sequenz zusammen mit einer Auswertungsanfrage zum Auffinden von CNVs in der Ziel-Genom-DNA-Sequenz zu empfangen, wobei die Auswertungsanfrage eine Vielzahl von charakteristischen Attributen umfasst, die sich auf die Ziel-Genom-DNA-Sequenz beziehen;
Vergleichen der Vielzahl von charakteristischen Attributen in der Auswertungsanfrage mit vorab in der Datenbankanordnung (102) gespeicherten Metadaten, die jeder aus einer Vielzahl von Proben-Genom-DNA-Sequenzen zugeordnet sind, umfassend: Nehmen eines Datensatzes der Vielzahl von charakteristischen Attributen in der vom Benutzer empfangenen Auswertungsanfrage, Abrufen der Vielzahl von charakteristischen Attributen in den Metadaten aus der Datenbankanordnung und Durchführen eines Vergleichs mit der Vielzahl von charakteristischen Attributen in den Metadaten;
Nutzen des Kandidaten-Referenzpanels zum Auswählen des maßgeschneiderten Referenzpanels für die Ziel-Genom-DNA-Sequenz, und zwar auf der Grundlage des Vergleichs der Informationen in der Auswertungsanfrage mit den Metadaten jeder Proben-Genom-DNA-Sequenz und einer Vielzahl von definierten Kriterien, die prüfen, ob mindestens ein Protokoll, das angewendet wurde, um die Proben-Genom-DNA-Sequenz abzuleiten, mit dem mindestens einen Protokoll übereinstimmt, das angewendet wurde, um die Ziel-Genom-DNA-Sequenz abzuleiten; und
Nutzen des maßgeschneiderten Referenzpanels, das die identifizierte Menge von Proben-Genom-DNA-Sequenzen umfasst, zum Auffinden von CNVs in der Ziel-Genom-DNA-Sequenz, wobei die Benutzerschnittstelle (106) dafür konfiguriert ist, die Eingabe der Ziel-Genom-DNA-Sequenz separat zu einem Zeitpunkt zu ermöglichen, der sich von einem Zeitpunkt unterscheidet, zu dem das Referenzpanel identifiziert und zur Verwendung als Referenzpanel für die Ziel-Genom-DNA-Sequenz festgelegt wurde.

12. Verfahren nach Anspruch 11, wobei das Verfahren umfasst: Nutzen einer CNV-Ermittlungsanwendung durch Verwendung der Computeranordnung (104) zum Auffinden von CNVs in der Ziel-Genom-DNA-Sequenz, und wobei mindestens eine CNV-Ermittlungsanwendung in der Datenbankanordnung (102) gespeichert ist.

13. Verfahren nach einem der Ansprüche 11 oder 12, wobei Prüfen, ob mindestens ein Protokoll, das angewendet wurde, um die Proben-Genom-DNA-Sequenz abzuleiten, mit dem mindestens einen Protokoll übereinstimmt, das angewendet wurde, um die Ziel-Genom-DNA-Sequenz abzuleiten, umfasst: Prüfen, ob:
eine Art der Probe, die verwendet wurde, um die Proben-Genom-DNA-Sequenz abzuleiten, mit der Art der Probe übereinstimmt, die verwendet wurde, um die Ziel-DNA-Sequenz abzuleiten;
das Geschlecht der Person, von der die Probe für die Proben-Genom-DNA-Sequenz erlangt wurde, mit dem Geschlecht der Person übereinstimmt, von der die Probe für die Ziel-Genom-DNA-Sequenz erlangt wurde; und/oder
die Familienanamnese der Person, von der die Proben-Genom-DNA-Sequenz erlangt wurde, sich von der Familienanamnese der Person unterscheidet, von der die Ziel-Genom-DNA-Sequenz erhalten wurde.

14. Computerprogrammprodukt, umfassend ein nichtflüchtiges, computerlesbares Speichermedium, auf dem computerlesbare Anweisungen gespeichert sind, wobei die computerlesbaren Anweisungen durch eine computergestützte Vorrichtung ausführbar sind, die Verarbeitungshardware umfasst, um ein Verfahren nach einem der Ansprüche 11 bis 12 auszuführen.

## Revendications

1. Système de détection d'erreurs de variant de nombre de copies (CNV) en utilisant un panel de référence personnalisé, dans lequel le système comprend :
un agencement de base de données (102) qui est configuré pour stocker une pluralité de séquences d'ADN génomique d'échantillon et des métadonnées qui sont associées à chaque séquence d'ADN génomique d'échantillon de la pluralité de séquences d'ADN génomique d'échantillon, dans lequel la pluralité de séquences d'ADN génomique d'échantillon comprend des séquences pré-enregistrées générées à partir d'exécutions de séquenceur passées et récentes ;
un agencement informatique (104) qui est couplé en communication à l'agencement de base de données (102), dans lequel l'agencement informatique (104) est configuré pour :
acquérir la pluralité de séquences d'ADN génomique d'échantillon à partir de l'agencement de base de données (102) ;
récupérer une pluralité d'attributs caractéristiques liés aux séquences d'ADN génomique d'échantillon pour générer les métadonnées, dans lequel la pluralité d'attributs caractéristiques liés à chacune des séquences d'ADN génomique d'échantillon comprend au moins un protocole appliqué pour dériver une séquence d'ADN génomique, dans lequel le au moins un protocole comprend la sélection :
d'un type de séquençage pour la dérivation de la séquence d'ADN génomique ;
d'une zone d'intérêt génomique pour la dérivation de la séquence d'ADN génomique ;
d'un type d'échantillon utilisé pour obtenir la séquence d'ADN génomique ;
du sexe d'un individu à partir duquel l'échantillon est acquis pour la dérivation de la séquence d'ADN génomique ; et/ou
des antécédents familiaux de l'individu duquel l'échantillon est acquis pour la dérivation de la séquence d'ADN génomique ;
étiqueter les métadonnées qui comprennent la pluralité d'attributs caractéristiques avec chaque séquence d'ADN génomique d'échantillon de la pluralité de séquences d'ADN génomique d'échantillon ;
stocker la pluralité des séquences d'ADN génomique d'échantillon et les métadonnées associées à chaque séquence d'ADN génomique d'échantillon de la pluralité de séquences d'ADN génomique d'échantillon dans l'agencement de base de données (102) ;
identifier les séquences d'ADN génomique d'échantillon présentant les mêmes métadonnées parmi la pluralité de séquences d'ADN génomique d'échantillon ;
regrouper les séquences d'ADN génomique d'échantillon identifiées présentant les mêmes métadonnées en un groupe commun ;
stocker chaque groupe de séquences d'ADN génomique d'échantillon identifiées présentant les mêmes métadonnées en tant que projet d'une pluralité de projets ;
étiqueter chaque projet de la pluralité de projets avec les métadonnées des séquences d'ADN génomique d'échantillon présentes dans ce projet, dans lequel la pluralité de projets présentant les séquences d'ADN génomique d'échantillon forme un panel de référence candidat ;
rendre une interface utilisateur (106) qui est configurée pour recevoir une séquence d'ADN génomique cible conjointement avec une demande d'interprétation pour appeler des CNV dans la séquence d'ADN génomique cible, dans lequel la demande d'interprétation comprend une pluralité d'attributs caractéristiques liés à la séquence d'ADN génomique cible ;
comparer la pluralité d'attributs caractéristiques dans la demande d'interprétation avec les métadonnées préstockées associées à chaque séquence d'ADN génomique d'échantillon de la pluralités de séquences d'ADN génomique d'échantillon dans l'agencement de base de données (102), comprenant la prise d'un enregistrement de la pluralité d'attributs caractéristiques dans la demande d'interprétation reçue de l'utilisateur, la récupération de la pluralité d'attributs caractéristiques dans les métadonnées à partir de l'agencement de base de données et l'exécution d'une comparaison avec la pluralité d'attributs caractéristiques dans les métadonnées ;
utiliser le panel de référence candidat pour sélectionner le panel de référence personnalisé pour la séquence d'ADN génomique cible, sur la base de la comparaison des informations contenues dans la demande d'interprétation avec les métadonnées de chaque séquence d'ADN génomique d'échantillon et une pluralité de critères définis qui vérifient si au moins un protocole appliqué pour dériver la séquence d'ADN génomique d'échantillon correspond à le au moins un protocole appliqué pour dériver la séquence d'ADN génomique cible ; et
utiliser le panel de référence personnalisé comprenant l'ensemble identifié de séquences d'ADN génomique d'échantillon pour appeler des CNV dans la séquence d'ADN génomique cible, dans lequel l'interface utilisateur (106) est configurée pour permettre la soumission d'une séquence d'ADN génomique cible séparément à un moment qui est différent de celui auquel le panel de référence est identifié et spécifié pour être utilisé comme panel de référence pour la séquence d'ADN génomique cible.

2. Système selon la revendication 1, dans lequel la pluralité d'attributs caractéristiques liés à la pluralité de séquences d'ADN génomique d'échantillon dans les métadonnées et la pluralité d'attributs caractéristiques liés à la séquence d'ADN génomique cible dans la demande d'interprétation sont mutuellement communs.

3. Système selon l'une quelconque des revendications 1 et 2, dans lequel l'agencement informatique (104) est en outre configuré pour rejeter la demande d'interprétation, si un nombre de séquences d'ADN génomique d'échantillon dans l'ensemble de séquences d'ADN génomique d'échantillon identifiées comme panel de référence est inférieur à un nombre spécifié de séquences d'ADN génomique d'échantillon.

4. Système selon l'une quelconque des revendications précédentes, dans lequel l'agencement informatique (104) est en outre configuré pour enregistrer le sexe de l'individu à partir duquel un échantillon est acquis pour dériver la séquence d'ADN génomique cible comme étant féminin, si le sexe de l'individu n'est pas divulgué dans la demande d'interprétation.

5. Système selon l'une quelconque des revendications précédentes, dans lequel l'agencement de base de données (102) est configuré pour stocker au moins une application de détection de CNV, et dans lequel l'agencement informatique (104) est configuré pour utiliser l'application de détection de CNV pour appeler des CNV dans la séquence d'ADN génomique cible.

6. Système selon la revendication 5, dans lequel l'agencement de calcul (104) est en outre configuré pour exécuter l'application de détection de CNV pour comparer une profondeur de lecture agrégée qui correspond à l'ensemble des séquences d'ADN génomique d'échantillon identifiées comme le panel de référence avec une profondeur de lecture correspondante de la séquence d'ADN génomique cible pour identifier des régions de la séquence d'ADN génomique cible qui chevauchent l'ensemble des séquences d'ADN génomique d'échantillon, indiquant une couverture de séquence supérieure à un niveau seuil.

7. Système selon la revendication 6, dans lequel l'agencement informatique (104) est en outre configuré pour exécuter l'application de détection de CNV pour :
- classer chaque séquence d'ADN génomique d'échantillon de l'ensemble des séquences d'ADN génomique d'échantillon dans le panel de référence, sur la base des régions identifiées dans la séquence d'ADN génomique cible qui chevauchent une ou plusieurs parties de chacune de l'ensemble de séquences d'ADN génomique d'échantillon ; et
- éliminer la séquence d'ADN génomique d'échantillon de l'ensemble des séquences d'ADN génomique d'échantillon du panel de référence présentant des régions de chevauchement inférieures au seuil de 50 %.

8. Système selon l'une quelconque des revendications 5 à 7, dans lequel l'agencement informatique (104) est en outre configuré pour exécuter l'application de détection de CNV pour générer un score de confiance comme mesure de précision dans l'appel de CNV dans la séquence d'ADN génomique cible.

9. Système selon l'une quelconque des revendications 2 à 8, dans lequel l'agencement informatique (104) est en outre configuré pour afficher des informations de patient via l'interface utilisateur, et dans lequel les informations de patient comprennent au moins des informations générales de patient et des informations de variant, et dans lequel :
les informations générales de patient comprennent :
un statut de la demande d'interprétation, dans lequel le statut de la demande d'interprétation est l'un quelconque des suivants : en attente, terminée, rejetée ;
un protocole appliqué pour dériver la séquence d'ADN génomique cible d'un patient ;
un type d'échantillon utilisé pour dériver la séquence d'ADN génomique cible du patient ; et
un panel de référence sélectionné pour appeler des CNV dans la séquence d'ADN génomique cible lorsque la demande d'interprétation est acceptée ; et
les informations de variant d'un patient comprennent :
un gain de CNV ou une perte de CNV dans la séquence d'ADN génomique cible par rapport à l'ensemble de séquences d'ADN génomique identifiées comme panel de référence ; et
un score de confiance généré pour l'appel des CNV dans la séquence d'ADN génomique cible.

10. Système selon une quelconque revendication précédente, dans lequel la vérification pour savoir si au moins un protocole appliqué pour dériver la séquence d'ADN génomique d'échantillon correspond à le au moins un protocole appliqué pour dériver la séquence d'ADN génomique cible comprend la vérification pour savoir si :
le type d'échantillon utilisé pour dériver la séquence d'ADN génomique d'échantillon correspond au type d'échantillon utilisé pour dériver la séquence d'ADN génomique cible ;
le sexe de l'individu duquel l'échantillon pour la séquence d'ADN génomique d'échantillon est acquis, correspond au sexe de l'individu duquel l'échantillon pour la séquence d'ADN génomique cible est acquis ; et/ou
les antécédents familiaux de l'individu duquel la séquence d'ADN génomique d'échantillonnée est obtenue, sont différents des antécédents familiaux de l'individu duquel la séquence d'ADN génomique cible est obtenue.

11. Procédé de détection d'erreurs de variant de nombre de copies (CNV) en utilisant un panel de référence personnalisé, dans lequel le procédé est mis en œuvre à l'aide d'un système qui comprend un agencement de base de données (102) qui est configuré pour stocker une pluralité de séquences d'ADN génomique d'échantillon et des métadonnées qui sont associées à chaque séquence d'ADN génomique d'échantillon de la pluralité de séquences d'ADN génomique d'échantillon, dans lequel la pluralité de séquences d'ADN génomique d'échantillon comprennent des séquences pré-enregistrées générées à partir d'exécutions de séquenceur passées et récentes et un agencement informatique (104) qui est couplé en communication à l'agencement de base de données (102), le procédé comprenant :
l'acquisition, par l'utilisation de l'agencement informatique (104), de la pluralité de séquences d'ADN génomique d'échantillon à partir de l'agencement de base de données (102) ;
la récupération, par l'utilisation de l'agencement informatique (104), d'une pluralité d'attributs caractéristiques liés aux séquences d'ADN génomique de l'échantillon pour générer les métadonnées, dans lequel la pluralité d'attributs caractéristiques liés à chacune des séquences d'ADN génomique de l'échantillon comprend au moins un protocole appliqué pour dériver une séquence d'ADN génomique, dans lequel le au moins un protocole comprend la sélection :
d'un type de séquençage pour la dérivation de la séquence d'ADN génomique ;
d'une zone d'intérêt génomique pour la dérivation de la séquence d'ADN génomique ;
d'un type d'échantillon utilisé pour une dérivation de la séquence d'ADN génomique ;
du sexe d'un individu à partir duquel l'échantillon est acquis pour la dérivation de la séquence d'ADN génomique ; et
des antécédents familiaux de l'individu duquel l'échantillon est acquis pour la dérivation de la séquence d'ADN génomique ;
l'étiquetage, par l'utilisation de l'agencement informatique (104), des métadonnées qui comprennent la pluralité d'attributs caractéristiques avec chaque séquence d'ADN génomique d'échantillon de la pluralité de séquences d'ADN génomique d'échantillon ;
le stockage, par l'utilisation de l'agencement informatique (104), de la pluralité de séquences d'ADN génomique d'échantillon et des métadonnées associées à chaque séquence d'ADN génomique d'échantillon de la pluralité de séquences d'ADN génomique d'échantillon dans l'agencement de base de données (102) ;
l'identification des séquences d'ADN génomique d'échantillon présentant les mêmes métadonnées parmi la pluralité de séquences d'ADN génomique d'échantillon ;
le regroupement des séquences d'ADN génomique d'échantillon identifiées présentant les mêmes métadonnées en un groupe commun ;
le stockage de chaque groupe de séquences d'ADN génomique d'échantillon identifiées présentant les mêmes métadonnées en tant que projet d'une pluralité de projets ;
l'étiquetage de chaque projet de la pluralité de projets avec les métadonnées des séquences d'ADN génomique d'échantillon présentes dans ce projet, dans lequel la pluralité de projets présentant les séquences d'ADN génomique d'échantillon forme un panel de référence candidat ; et
le rendu, par l'utilisation de l'agencement informatique (104), d'une interface utilisateur (106) configurée pour recevoir une séquence d'ADN génomique cible conjointement avec une demande d'interprétation pour appeler des CNV dans la séquence d'ADN génomique cible, dans lequel la demande d'interprétation comprend une pluralité d'attributs caractéristiques liés à la séquence d'ADN génomique cible ;
la comparaison de la pluralité d'attributs caractéristiques dans la demande d'interprétation avec des métadonnées associées à chaque séquence d'ADN génomique d'échantillon d'une pluralité de séquences d'ADN génomique d'échantillon préstockées dans l'agencement de base de données (102), comprenant la prise d'un enregistrement de la pluralité d'attributs caractéristiques dans la demande d'interprétation reçue de l'utilisateur, la récupération de la pluralité d'attributs caractéristiques dans les métadonnées à partir de l'agencement de base de données et l'exécution d'une comparaison avec la pluralité d'attributs caractéristiques dans les métadonnées ;
l'utilisation du panel de référence candidat pour sélectionner le panel de référence personnalisé pour la séquence d'ADN génomique cible, sur la base de la comparaison des informations contenues dans la demande d'interprétation avec les métadonnées de chaque séquence d'ADN génomique d'échantillon et une pluralité de critères définis qui vérifient si au moins un protocole appliqué pour dériver la séquence d'ADN génomique d'échantillon correspond à le au moins un protocole appliqué pour dériver la séquence d'ADN génomique cible ; et
l'utilisation du panel de référence comprenant l'ensemble identifié de séquences d'ADN génomique d'échantillon pour appeler des CNV dans la séquence d'ADN génomique cible, dans lequel l'interface utilisateur (106) est configurée pour permettre la soumission d'une séquence d'ADN génomique cible séparément à un moment qui est différent de celui auquel le panel de référence est identifié et spécifié pour être utilisé comme panel de référence pour la séquence d'ADN génomique cible.

12. Procédé selon la revendication 11, dans lequel le procédé comprend l'utilisation, par l'utilisation de l'agencement informatique (104), d'une application de détection de CNV pour l'appel de CNV dans la séquence d'ADN génomique cible, et dans lequel au moins une application de détection de CNV est stockée dans le dispositif de base de données (102).

13. Procédé selon l'une quelconque des revendications 11 ou 12, dans lequel la vérification pour savoir si au moins un protocole appliqué pour dériver la séquence d'ADN génomique d'échantillon correspond à le au moins un protocole appliqué pour dériver la séquence d'ADN génomique cible comprend la vérification pour savoir si :
le type d'échantillon utilisé pour dériver la séquence d'ADN génomique d'échantillon correspond au type d'échantillon utilisé pour dériver la séquence d'ADN génomique cible ;
le sexe de l'individu duquel l'échantillon pour la séquence d'ADN génomique d'échantillon est acquis, correspond au sexe de l'individu duquel l'échantillon pour la séquence d'ADN génomique cible est acquis ; et/ou
les antécédents familiaux de l'individu duquel la séquence d'ADN génomique d'échantillonnée est obtenue, sont différents des antécédents familiaux de l'individu duquel la séquence d'ADN génomique cible est obtenue.

14. Produit de programme d'ordinateur comprenant un support de stockage non transitoire lisible par ordinateur sur lequel des instructions lisibles par ordinateur sont stockées, les instructions lisibles par ordinateur étant exécutables par un dispositif informatisé comprenant un matériel de traitement pour exécuter un procédé tel que revendiqué dans l'une quelconque des revendications 11 à 12.
